# EUROPEAN PATENT APPLICATION

(11) **EP 2 177 496 A1**
(43) Date of publication of application: **21.04.2010**
(21) Application number: 08305646.5
(22) Date of filing: 07.10.2008
(51) Int. Cl.: C07C 41/01, C07C 43/23

(54) **New analogs of polysphorin and uses thereof for treating or preventing malaria**

(71) Applicant: Assistance Publique - Hôpitaux de Paris, 75004 Paris (FR); Centre National de la Recherche Scientifique, 75016 Paris (FR); UNIVERSITE PIERRE ET MARIE CURIE, 75005 Paris (FR); Université Louis Pasteur, 67000 Strasbourg (FR)
(72) Inventor: Choppin, Sabine, 67370 Wollenheim (FR); Colobert, Françoise, 67450 Lampertheim (FR); Hanquet, Gilles, 67210 Obernai (FR); Leroux, Frédéric, 67850 Herrlisheim (FR); Mahmoudi, Nassira, 77340 Pontault-Combault (FR); Mazier, Dominique, 75010 Paris (FR)
(74) Representative: Gallois, Valérie

(57) **Abstract**

The present invention discloses new analogs of polysphorin and their uses as antimalarial drugs which are highly effective in the hepatic phase of the disease. The present invention further discloses a new method for preparing these compounds.

## Description

### Field of the Invention

The present invention relates to new analogs of polysphorin, to a method of preparation of polysphorin analogs and the use of polysphorin analogs for preventing or treating malaria.

### Background of the Invention

Malaria is one of the most common infectious diseases and an enormous public health problem. It is widespread in tropical and subtropical regions, including parts of the Americas, Asia, and Africa. Each year, it causes disease of approximately 515 million people and kills between one and three million people. The disease is caused by protozoan parasites of the genus *Plasmodium.* Only four types of the *Plasmodium* parasite can infect humans; the most serious forms of the disease are caused by *P. falciparum* and *P. vivax*, but other related species (*P. ovale, P. malariae*) can also affect humans.

In endemic areas, mortality due to malaria clearly increased and the resistance of the parasite to the usual drugs (chloroquinine, mefloquinine) is regarded as one of the major factors contributing to this aggravation.

For instance, chloroquine is very cheap and, until recently, was very effective. However, resistance of *P. falciparum* to chloroquine has spread from Asia to Africa, making the drug ineffective against the most dangerous *Plasmodium* strain in many affected regions of the world. Unfortunately, chloroquine-resistance is associated with reduced sensitivity to other drugs such as quinine and amodiaquine.

The resistance of the parasite to the drugs depending on the geographical origin of the parasite led WHO to propose a combination of drugs with various activities. The artemisinin-based combinations are the best drugs currently available for the treatment of the malaria due to *P. falciparum.* They will however not make it possible to control the malaria nor to slow down the development of resistance in endemic zone for various reasons: their relatively high cost; the limited knowledge of the health personnel and the public of the concerned countries concerning the concept of the combined therapies; and, the lack of data on safety of the artemisinin-based combinations during the pregnancy.

The malaria prevention rests on two types of measures, the preventive treatment with certain drugs and a set of precautions aiming at limiting the risks of infections. Use of prophylactic drugs is seldom practical for full-time residents of malaria-endemic areas, and their use is usually restricted to short-term visitors and travelers to malarial regions. This is due to the cost of purchasing the drugs, negative side effects from long-term use, and because some effective anti-malarial drugs are difficult to obtain outside of wealthy nations.

Treatment of malaria is complicated by the resistances to different antimalarial drugs, with variable frequencies and intensities according to areas of transmission.

In humans, malaria develops via two phases: an hepatic phase and an erythrocytic phase. When an infected mosquito takes a blood meal, sporozoites in the mosquito's saliva enter the bloodstream and migrate to the liver. Then, they infect hepatocytes where they multiply asexually and asymptomatically. Once in the liver, they differentiate to yield thousands of merozoites which, following rupture of their host cells, escape into the blood and infect red blood cells, thus beginning the erythrocytic stage of the life cycle. Within the red blood cells, the parasites further multiply asexually, periodically breaking out of their hosts to invade fresh red blood cells. Some *P. vivax* and *P. ovale* sporozoites do not immediately develop into erythrocytic-phase merozoites, but instead produce hypnozoites responsible for long incubation and late relapses.

Most of the research programs were focused on the erythrocytic stage of *P. falciparum*, which causes the potentially mortal malarial crisis. However, before multiplying in blood (process responsible for pathogenesis), the parasite multiplies in the liver where it could be a target of drug. Today, only two drugs, the primaquine and the tafenoquine, are active during the hepatic phase of parasite development. However, these drugs have a hematologic toxicity and their use is limited, in particular in Africa, because of their cost and of the frequency of the subjects presenting a G6PD deficit. Atovaquone is another drug having a hepatic activity. However, this molecule does not have any activity on the hypnozoïtes and there is a high natural frequency of cytochrome B mutants which leads to a high failure rate when atovaquone is used on its own to treat malaria.

In addition, an important issue is the elimination of dormant forms of parasites (i. e., hypnozoïtes) in liver in order to reach a complete recovery.

The development of drugs able to inhibit the parasite during the liver phase is interesting for three main reasons. Firstly, these drugs could act as prophylactic agents by preventing the parasite from reaching the blood. Secondly, the probability of any given mutation being present in liver-stage parasites is many orders of magnitude smaller than the same mutation being found in blood stages. Thirdly, they could act against relapse and constitute a radical cure of the parasite.

Accordingly, there is a strong need of antimalarial drugs active during the hepatic phase of parasite development, and in particular on the hypnozoites, without any or weak toxicity.

### Summary of the Invention

The present invention provides new antimalarial drugs which are highly effective in the hepatic phase with low toxicity and very low cost. In particular, the present invention further provides drugs active on the hepatic phases and, optionally, at the erythrocytic phase.

In a first aspect, the present invention provides new polysphorin analogs having the formula (A) : 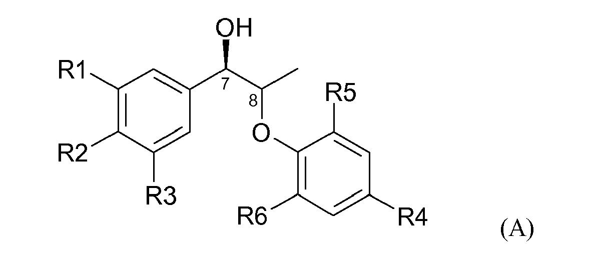 wherein
R1 and R2 are selected from the group consisting of OMe, OCF₃, OCHF₂, OCH₂F, Me, CF₃, CHF₂, CH₂F, and F, or together form -O-CH₂-O-, -O-CHF-O-, or -O-CF₂-O-,
R3 is selected from the group consisting of H, OMe, OCF₃, OCHF₂, OCH₂F, Me, CF₃, CHF₂, CH₂F and F,
R5 and R6 are selected from the group consisting of OMe, OCF₃, OCHF₂, OCH₂F, Me, CF₃, CHF₂, CH₂F and F, and
R4 is selected from the group consisting of H, a C₁-C₆ alkyl, and a phenyl optionally substituted by one to three substituents selected in the group consisting of a C₁-C₃ alkyl or alkoxy, halogens, hydroxyl, cyano, amino, and nitro,
or a pharmaceutically acceptable salt thereof.

Preferably, R1 and R2 are both OMe or together form -O-CH₂-O-, -O-CHF-O-, or -O-CF₂-O-, R3 is H or OMe, R5 and R6 are OMe, and R4 is selected from the group consisting of H, a C₁-C₆ alkyl and a phenyl, more preferably from the group consisting of H, a C₁-C₃ alkyl and a phenyl.

In a first preferred embodiment, R4 is H, a methyl or a phenyl. More preferably, R4 is a methyl or a phenyl. Preferably, R1, R2 and R3 are OMe. Alternatively, R1 and R2 together form -O-CH₂-O-, -O-CHF-O-, or -O-CF₂-O-, and R3 is H. In a preferred embodiment, the conformation between C7 and C8 is *syn,* and R4 is H or methyl, preferably a methyl.

In a second aspect, the present invention concerns a pharmaceutical composition comprising a new polysphorin analog according to the invention and, optionally, a pharmaceutically acceptable carrier and/or excipient. The pharmaceutical composition can further comprise one or several additional antimalarial drugs or compounds that treat one or several of malaria symptoms.

In a third aspect, the present invention concerns a new polysphorin analog according to the invention as described above as a medicament, preferably as antimalarial prophylactic or therapeutic agent. In particular, the present invention concerns a new polysphorin analog according to the invention as antimalarial prophylactic or therapeutic agent at the hepatic stage or at both hepatic and erythrocytic stages.

In a fourth aspect, the present invention concerns a method for preventing or treating malaria in a subject comprising administering a therapeutically effective amount of a new polysphorin analog according to the invention to said subject.

In fifth aspect, the present invention concerns a polysphorin or a polysphorin analog as antimalarial prophylactic or therapeutic agent at the hepatic stage.

In another aspect, the present invention also concerns a method for preparing polysphorin analogs (i.e., Ar1-CHOH-CHMe-O-Ar2), comprising:
a) substituting the bromide of a first aromatic compound (Arl-Br) by a propylacyl group (-CO-CH₂-CH₃), thereby preparing the corresponding aryl ketone Ar1-CO-CH₂-CH₃;
b) introducing a bromide on the carbon atom in alpha of the carbonyl group in the propylacyl rest, thereby preparing the corresponding bromo aryl ketone Ar1-CO-CHBr-CH₃;
c) substituting the bromide by an ether derivative of a second aromatic compound (Ar²-OH), thereby preparing the corresponding aryl ketone ether Ar1-CO-CHMe-OAr2; and
d) reducing the compound obtained from step c), thereby obtaining a polysphorin analog (i.e., Ar1-CHOH-CHMe-O-Ar2).

### Detailed description of the Invention

### Definition:

Whenever within this whole specification "treatment or prevention of malaria", "for treating or preventing malaria" or the like is mentioned with reference to polysphorin, a polysphorin analog or a new polysphorin analog, there is meant:
a) a method of treatment (=for treating) of malaria, said method comprising the step of administering polysphorin or an analog thereof, (preferably in a pharmaceutically acceptable carrier material) to a subject, especially a human, in need of such treatment, in a dose that allows for the treatment of said disease (=a therapeutically effective amount), preferably in a dose (amount) as specified to be preferred hereinabove and hereinbelow;
b) the use of polysphorin or an analog thereof for the treatment or the prevention of malaria; or polysphorin or an analog thereof for use in said treatment or prevention of malaria (especially in a human); or polysphorin or an analog thereof for the treatment or the prevention of malaria;
c) the use of polysphorin or an analog thereof, for the manufacture of a pharmaceutical preparation for the treatment or prevention of malaria; and/or
d) a pharmaceutical preparation comprising a dose of polysphorin or an analog thereof that is appropriate for the treatment or prevention of malaria; or any combination of a), b), c) and d), in accordance with the subject matter allowable for patenting in a country where this application is filed;
e) a method of using polysphorin or an analog thereof for the manufacture of a pharmaceutical preparation for the treatment or prevention of malaria, comprising admixing said polysphorin or an analog thereof with a pharmaceutically acceptable carrier.

In cases where "malaria at the hepatic stage", "malaria at the erythrocytic stage" or "asymptomatic malaria" are mentioned instead of "malaria", categories a) to e) are also encompassed, meaning that the respective malaria stage can be filled in under a) to e) above instead of "malaria", in accordance with the patentable subject matter.

The term "malaria" includes the art recognized condition known as "malaria" e.g. disorders which are caused by a protozoan of the genus *Plasmodium.* Malaria is generally characterized by symptoms such as headache, malaise, anemia, splenomegaly, and paroxyms with cold, hot, and wet stages and is transmitted by mosquitoes. In a further embodiment, the protozoan is selected from the group consisting of : *P. falciparum, P. vivax, P. ovale,* and *P. malariae*.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more prophylactic or therapeutic agents to a patient with such a disease.

In particular, the term "to treat malaria" or "treatment of malaria" refers to the eradication of malaria or to the diminishing or alleviation of at least one symptom of malaria, such as body aches, malaise, nausea, vomiting, anemia, splenomegaly, and/or fever. In certain embodiments, this term also refers to the prevention or treatment of severe malaria occurring when *Plasmodium* infections are complicated by serious organ failures or abnormalities in the patient's blood or metabolism.

As used herein, the term "prevention", "prevent" or "preventing" refers to the prevention of the onset, recurrence, or spread of the disease in a patient resulting from the administration of a prophylactic or therapeutic agent.

In particular, the term "to prevent malaria" or "prevention of malaria" refers to limiting the risks of infections or preventing the parasite from reaching the blood of the subject. It includes administration of a polysphorin analog of the invention to a subject who is not currently suffering from malaria, such that the subject does not contract malaria for a period of time after the administration and after exposure to malaria.

A "subject" or a "patient" is any mammal, preferably a human being. The human being can be a human infant, child, adolescent or adult.

The term "pharmaceutically acceptable salt" refers to salts which are non-toxic for a patient and suitable for maintaining the stability of a therapeutic agent and allowing the delivery of said agent to target cells or tissue. Pharmaceutically acceptable salts are well known in the art (Berge et al., 1977).

By "C₁-C₆ alkyl" is intended a saturated alkyl, in particular methyl, ethyl, propyl, isopropyl, butyl, isobutyl, *tert*-butyl, pentyl, hexyl and the other isomeric forms thereof. By "C₁-C₃ alkyl" is intended methyl, ethyl, propyl, and isopropyl. The term "Me" refers to a methyl group.

By "C₂-C₆ alkenyl" is intended a C₂-C₆ alkyl having at least one unsaturated ethylene bond and including for instance a ethenyl, a propenyl (1-propenyl or 2-propenyl), a 1- or 2- methylpropenyl, a butenyl (1-butenyl, 2-butenyl, or 3-butenyl), a methylbutenyl, a 2-ethylpropenyl, a pentenyl (1-pentenyl, 2-pentenyl, 3-pentenyl, 4-pentenyl), an hexenyl (1-hexenyl, 2-hexenyl, 3-hexenyl, 4-hexenyl, 5-hexenyl), and the other isomeric forms thereof. By "C₂-C₃ alkenyl" is intended a C₂-C₃ alkyl having at least one unsaturated ethylene bond and including for instance a ethenyl, a propenyl such as (E)-propenyl and allyl.

By "C₁-C₃ alkoxy" is intended methoxy, ethoxy, propyloxy, and isopropyloxy.
The term "OMe" refers to a methoxy group (-O-CH₃).

### The present invention concerns new polysphorin analogs having the formula (A)

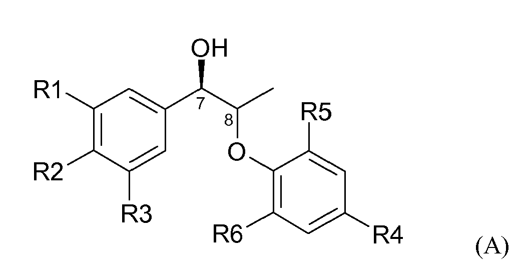

wherein
R1 and R2 are selected from the group consisting of OMe, OCF₃, OCHF₂, OCH₂F, Me, CF₃, CHF₂, CH₂F and F, or together form -O-CH₂-O-, -O-CHF-O-, or -O-CF₂-O-,
R3 is selected from the group consisting of H, OMe, OCF₃, OCHF₂, OCH₂F, Me, CF₃, CHF₂, CH₂F and F,
R5 and R6 are selected from the group consisting of OMe, OCF₃, OCHF₂, OCH₂F, Me, CF₃, CHF₂, CH₂F and F, and
R4 is selected from the group consisting of H, a C₁-C₆ alkyl and a phenyl optionally substituted by one to three substituents selected in the group consisting of a C₁-C₃ alkyl or alkoxy, halogens, hydroxyl, cyano, amino, and nitro,
or a pharmaceutically acceptable salt thereof.

In a first particular embodiment of the polysphorin analog of formula (A), R1 and R2 are both OMe, OCF₃, OCHF₂, OCH₂F, Me, CF₃, CHF₂, CH₂F or F, preferably OMe, OCF₃, OCHF₂, OCH₂F, or F, more preferably OMe or F, and R3 is H or identical to R1 and R2. Alternatively, R1 and R2 together form -O-CH₂-O- or -O-CF₂-O-, and R3 is H.

In a second particular embodiment of the polysphorin analog of formula (A), R5 and R6 are both OMe, OCF₃, OCHF₂, OCH₂F, Me, CF₃, CHF₂, CH₂F or F, preferably OMe, OCF₃, OCHF₂, OCH₂F, CF₃ or F, more preferably OMe or F.

In a third particular embodiment of the polysphorin analog of formula (A), R4 is selected from the group consisting of H, a C₁-C₃ alkyl and a phenyl, more preferably from the group consisting of H, a methyl and a phenyl.

In a fourth particular embodiment of the polysphorin analog of formula (A), R1 and R2 are both OMe, OCF₃, OCHF₂, OCH₂F, Me, CF₃, CHF₂, CH₂F or F, preferably OMe, OCF₃, OCHF₂, OCH₂F, or F, more preferably OMe or F, R3 is H or identical to R1 and R2, and R5 and R6 are both OMe, OCF₃, OCHF₂, OCH₂F, Me, CF₃, CHF₂, CH₂F or F, more preferably OMe, OCF₃, OCHF₂, OCH₂F, CF₃ or F. Preferably, R4 is selected from the group consisting of H, a C₁-C₃ alkyl and a phenyl, more preferably from the group consisting of H, a methyl and a phenyl.

In a fifth particular embodiment of the polysphorin analog of formula (A), R1 and R2 together form -O-CH₂-O-, -O-CHF-O-, or -O-CF₂-O-, R3 is H and R5 and R6 are both OMe, OCF₃, OCHF₂, OCH₂F, Me, CF₃, CHF₂, CH₂F or F, more preferably OMe, OCF₃, OCHF₂, OCH₂F, CF₃ or F. Preferably, R4 is selected from the group consisting of H, a C₁-C₃ alkyl and a phenyl, more preferably from the group consisting of H, a methyl and a phenyl.

In a sixth particular embodiment of the polysphorin analog of formula (A), R1 and R2 are both OMe, OCF₃, OCHF₂, OCH₂F, Me, CF₃, CHF₂, CH₂F or F, more preferably OMe or F, R3 is H or identical to R1 and R2, and R4 is selected from the group consisting of H, a C₁-C₃ alkyl and a phenyl, more preferably from the group consisting of H, a methyl and a phenyl.

In a seventh particular embodiment of the polysphorin analog of formula (A), R1 and R2 together form -O-CH₂-O-, -O-CHF-O-, or -O-CF₂-O-, R3 is H and R4 is selected from the group consisting of H, a C₁-C₃ alkyl and a phenyl, more preferably from the group consisting of H, a methyl and a phenyl.

In a eight particular embodiment of the polysphorin analog of formula (A), R5 and R6 are both OMe, OCF₃, OCHF₂, OCH₂F, Me, CF₃, CHF₂, CH₂F or F, more preferably OMe, OCF₃, OCHF₂, OCH₂F, CF₃ or F, and R4 is selected from the group consisting of H, a C₁-C₃ alkyl and a phenyl, more preferably from the group consisting of H, a methyl and a phenyl.

The conformation between C7 and C8 can be *syn* or *anti.* In a preferred embodiment, the conformation between C7 and C8 is *syn.*

In a preferred embodiment, the present invention concerns new polysphorin analogs having the formula (B) : 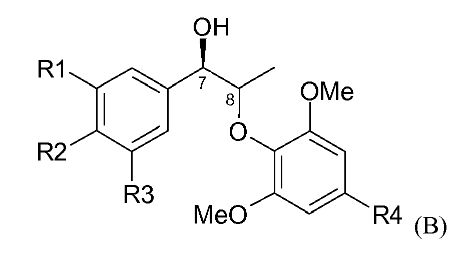 wherein
R1 and R2 are both OMe or together form -O-CH₂-O-, -O-CHF-O-, or -O-CF₂-O-,
R3 is H or OMe, and
R4 is selected from the group consisting of H, a C₁-C₆ alkyl and a phenyl.

In a first particularly preferred embodiment of the polysphorin analog of formula (B), R1, R2 and R3 are OMe.

In a second particularly preferred embodiment of the polysphorin analog of formula (B), R1 and R2 together form -O-CH₂-O- or -O-CF₂-O-, and R3 is H. Preferably, R1 and R2 together form -O-CF₂-O- and R3 is H.

In a third particularly preferred embodiment of the polysphorin analog of formula (B), R4 is selected from the group consisting of H, a C₁-C₃ alkyl and a phenyl, more preferably from the group consisting of H, a methyl and a phenyl. In a most preferred embodiment, R4 is a methyl or a phenyl.

In a more particularly preferred embodiment of the polysphorin analog of formula (B), R1 and R2 together form -O-CF₂-O-, R3 is H, and R4 is H, a methyl or a phenyl, preferably a methyl or a phenyl. In a further preferred embodiment of the polysphorin analog of formula (B), R1, R2 and R3 are OMe and R4 is H, a methyl or a phenyl, preferably a methyl or a phenyl. The conformation between C7 and C8 can be *syn* or *anti.* In a preferred embodiment, the conformation between C7 and C8 is *syn.*

Preferably, in formula (B), R1, R2 and R3 are OMe; and R4 is a phenyl.
According to this embodiment, the compound has the formula (C): 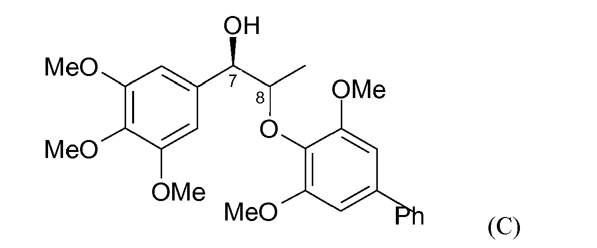 The compound of formula (C) can be *syn* or *anti.*

The present invention more specifically concerns new *syn* polysphorin analogs having the formula (D): 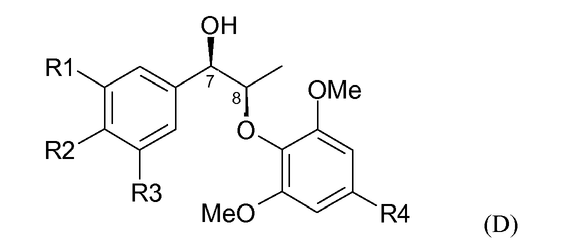 wherein R1 and R2 are both OMe or together form -O-CH₂-O-, -O-CHF-O-, or - O-CF₂-O-, R3 is H or OMe, R4 is selected from the group consisting of H, a C₁-C₆ alkyl and a phenyl, or any pharmaceutically acceptable salt thereof.

In a first preferred embodiment of the polysphorin analog of formula (D), R1, R2 and R3 are OMe.

In a second preferred embodiment of the polysphorin analog of formula (D), R1 and R2 together form -O-CH₂-O-, -O-CHF-O-, or -O-CF₂-O-, and R3 is H. Preferably, R1 and R2 together form -O-CF₂-O- and R3 is H.

In a third preferred embodiment of the polysphorin analog of formula (D), R4 is selected from the group consisting of H, a C₁-C₃ alkyl and a phenyl, more preferably from the group consisting of H, a methyl and a phenyl. In a most preferred embodiment, R4 is a methyl or a phenyl.

In a fourth preferred embodiment of the polysphorin analog of formula (D), R1, R2 and R3 are OMe and R4 is selected from the group consisting of H, a C₁-C₃ alkyl and a phenyl, more preferably from the group consisting of H, a methyl and a phenyl.

In a fifth preferred embodiment of the polysphorin analog of formula (D), R1 and R2 together form -O-CH₂-O-, -O-CHF-O-, or -O-CF₂-O-, R3 is H, and R4 is selected from the group consisting of H, a C₁-C₃ alkyl and a phenyl, more preferably from the group consisting of H, a methyl and a phenyl.

In a more preferred embodiment, R1 and R2 together form -O-CF₂-O-, R3 is H, R4 is H or a methyl, preferably a methyl. In a further preferred embodiment, R1, R2 and R3 are OMe and R4 is H or a methyl, preferably a methyl.

In a particularly preferred embodiment, the *syn* polysphorin analog of formula (D) has R1 and R2 together forming -O-CF₂-O- , R3 being H and R4 being a methyl.

In another particularly preferred embodiment, the *syn* polysphorin analog of formula (D) has R1, R2 and R3 being OMe and R4 being a methyl.

The present invention concerns new polysphorin analogs of formulae A-D, as described above, as a medicament, in particular as antimalarial prophylactic or therapeutic drug. In particular, the present invention concerns a polysphorin analog of formulae A-D or any pharmaceutically acceptable salt thereof, as antimalarial prophylactic or therapeutic agent at the hepatic stage or at both hepatic and erythrocytic stages.

The present invention also concerns a pharmaceutical composition comprising a polysphorin analog of formulae A-D or any pharmaceutically acceptable salt thereof and a combination thereof. In particular, the combination can comprise a mixture of *syn* and *anti* configurations of a same analog. Alternatively, the combination can comprise several different analogs of formulae A-D.

The pharmaceutical composition comprises a pharmaceutically acceptable carrier and/or excipient. These excipients and/or carriers are chosen according to the form of administration as described above.

The pharmaceutical composition according to the invention is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

Possible pharmaceutical compositions include those suitable for oral, rectal, topical (including transdermal, buccal and sublingual), or parenteral (including subcutaneous, intramuscular, intravenous and intradermal) administration. For these formulations, conventional excipient can be used according to techniques well known by those skilled in the art.

The compositions for parenteral administration are generally physiologically compatible sterile solutions or suspensions which can optionally be prepared immediately before use from solid or lyophilized form. Adjuvants such as a local anesthetic, preservative and buffering agents can be dissolved in the vehicle and a surfactant or wetting agent can be included in the composition to facilitate uniform distribution of the active ingredient.

For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

In a preferred embodiment, the pharmaceutical composition of the invention is suitable for oral administration.

Pharmaceutical composition according to the invention may be formulated to release the active drug substantially immediately upon administration or at any predetermined time or time period after administration.

The pharmaceutical composition can also comprise one or several additional active compounds such as other antimalarial drugs or compounds that treat one or several of malaria symptoms such as headache, body aches, malaise, nausea, vomiting, anemia, splenomegaly, and/or fever. Other antimalarial drugs include but are not limited to, chloroquine, mefloquine, proguanil, chlorproguanil, trimethoprim, lumefantrine, atovaquone, pyrimethamine-sulfadoxine, pyrimethamine-dapsone, halofantrine, quinine, quinidine, amodiaquine, amopyroquine, sulphonamides, artemisinin, arteflene, artemether, artesunate, primaquine, pyronaridine, and combinations thereof.

In a preferred embodiment, the pharmaceutical composition according to the invention comprises a therapeutically effective amount of polysphorin analog of the invention.

The present invention also concerns a polysphorin analog of formulae A-D, or any pharmaceutically acceptable salt thereof, for preventing or treating malaria. Polysphorin analogs of formulae A-D can be used in combination with other antimalarial drugs or compounds that treat one or several of malaria symptoms. In this case, polysphorin analogs and the other active molecules (such as those disclosed above) can be administered simultaneously or consecutively, by the same route or different routes.

The present invention further concerns a method for preventing or treating malaria in a subject comprising administering a therapeutically effective amount of a polysphorin analog of formulae A-D, or any pharmaceutically acceptable salt thereof, to said subject.

By a "therapeutically effective amount" is intended an amount of polysphorin analog of the invention administered to a patient that is sufficient to provide a therapeutic or prophylactic benefit in the treatment or prevention of malaria or to delay or minimize symptoms associated with malaria. Further, a therapeutically effective amount with respect to a polysphorin analog of the invention means that amount of therapeutic agent alone, or in combination with other therapies, that provides a therapeutic benefit in the treatment or prevention of malaria. The amount of polysphorin analog of the invention to be administered will vary with the particular compound chosen (e.g. depending on the potency, efficacy, and half-life of the compound); the route of administration chosen; the nature of the *Plasmodium* parasite; the phase of the disease (liver or erythrocytic phase); the severity of the infection; the age, size, weight, and physical condition of the patient to be treated; the medical history of the patient to be treated; the duration of the treatment; the nature of concurrent therapy; the desired therapeutic effect; and like factors, but can nevertheless be routinely determined by the skilled artisan.

Preferably, the medicament is suitable for a dose regimen of from 1 to 15 mg/kg/day of the compound, more preferably of about 7 or 8 mg/kg/day.

Preferably, the medicament comprises a unit dose of from 10 mg to 2 g of the compound, more preferably of from 70 mg to 600 mg.

Polysphorin analogs of formulae A-D can be used to treat malaria at the hepatic phase and/or at the erythrocytic phase by inhibiting or eradicating the parasite in the liver and/or in the blood of the subject and/or by inhibiting or eradicating the dormant forms of parasites in liver.

The polysphorin analogs of formulae A-D can also be used as prophylactic agent by travelers to malaria-risk areas to prevent parasite infection caused by *Plasmodium* infected mosquito bites.

In addition of providing new polysphorin analogs of formulae A-D useful for treating malaria, the inventors surprisingly discovered that the polysphorin, in its *syn* and *anti* configuration, and in particular in its *syn* configuration, is effective against *Plasmodium* at the hepatic stage. This efficiency opens a novel way to treat malaria at the early and non-symptomatic stage, i.e. the hepatic stage. In addition, the polysphorin can also use as a prophylactic antimalarial agent. Accordingly, the present also concerns the polysphorin or a polysphorin analog of formula (E) for treating malaria at the hepatic stage, i.e. at the early non-symptomatic stage,
wherein the formula (E) is the following: 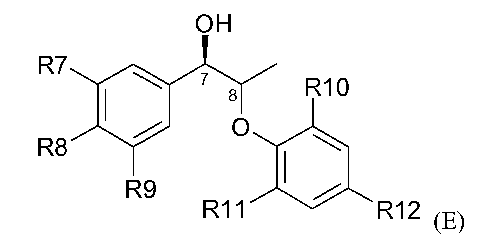 wherein
R7 and R8 are selected from the group consisting of OMe, OCF₃, OCHF₂, OCH₂F, Me, CF₃, CHF₂, CH₂F and F, or together form -O-CH₂-O-, -O-CHF-O-, or -O-CF₂-O-,
R9 is selected from the group consisting of H, OMe, OCF₃, OCHF₂, OCH₂F, Me, CF₃, CHF₂, CH₂F and F,
R10 and R11 are selected from the group consisting of OMe, OCF₃, OCHF₂, OCH₂F, Me, CF₃, CHF₂, CH₂F and F, and
R12 is selected from the group consisting of H, a C₁-C₆ alkyl, a C₂-C₆ alkenyl and a phenyl optionally substituted by one to three substituents selected in the group consisting of a C₁-C₃ alkyl or alkoxy, halogens, hydroxyl, cyano, amino, and nitro,
or any pharmaceutically acceptable salt thereof.

The present invention further concerns the use of polysphorin or a polysphorin analog of formula (E) for the manufacture of a medicament for treating the malaria at the hepatic stage, i.e. at the early non-symptomatic stage, or for preventing the malaria. In addition, it concerns a method for treating malaria in a subject at the hepatic phase, comprising administering a therapeutically effective amount of polysphorin or a polysphorin analog of formula (E).

In a first particular embodiment of the use and method of polysphorin or analogs thereof of formula (E), R7 and R8 are both OMe, OCF₃, OCHF₂, OCH₂F, Me, CF₃, CHF₂, CH₂F or F, preferably OMe, OCF₃, OCHF₂, OCH₂F or F, more preferably OMe or F, and R9 is H or identical to R7 and R8. Alternatively, R7 and R8 together form -O-CH₂-O- or -O-CF₂-O-, and R9 is H.

In a second particular embodiment of the use and method of polysphorin or analogs thereof of formula (E), R10 and R11 are both OMe, OCF₃, OCHF₂, OCH₂F, Me, CF₃, CHF₂, CH₂F or F, more preferably OMe, OCF₃, CF₃ or F.

In a third particular embodiment of the use and method of polysphorin or analogs thereof of formula (E), R12 is selected from the group consisting of H, a C₁-C₃ alkyl, a C₂-C₃ alkenyl and a phenyl, more preferably from the group consisting of H, a methyl, an allyl, a (E)-propenyl and a phenyl.

In a fourth particular embodiment of the use and method of polysphorin or analogs thereof of formula (E), R7 and R8 are both OMe, OCF₃, OCHF₂, OCH₂F, Me, CF₃, CHF₂, CH₂F or F, more preferably OMe or F, R9 is H or identical to R7 and R8, and R10 and R11 are both OMe, OCF₃, OCHF₂, OCH₂F, Me, CF₃, CHF₂, CH₂F or F, more preferably OMe, OCF₃, CF₃ or F. Preferably, R12 is selected from the group consisting of H, a C₁-C₃ alkyl and a phenyl, more preferably from the group consisting of H, a methyl and a phenyl.

In a fifth particular embodiment of the use and method of polysphorin or analogs thereof of formula (E), R7 and R8 together form -O-CH₂-O-, -O-CHF-O-, or -O-CF₂-O-, R9 is H and R10 and R11 are both OMe, OCF₃, OCHF₂, OCH₂F, Me, CF₃, CHF₂, CH₂F or F, more preferably OMe, OCF₃, CF₃ or F. Preferably, R12 is selected from the group consisting of H, a C₁-C₃ alkyl, a C₂-C₃ alkenyl and a phenyl, more preferably from the group consisting of H, a methyl, an allyl, a (E)-propenyl and a phenyl.

In a sixth particular embodiment of the use and method of polysphorin or analogs thereof of formula (E), R7 and R8 are both OMe, OCF₃, OCHF₂, OCH₂F, Me, CF₃, CHF₂, CH₂F or F, more preferably OMe or F, R9 is H or identical to R7 and R8, and R12 is selected from the group consisting of H, a C₁-C₃ alkyl, a C₂-C₃ alkenyl and a phenyl, more preferably from the group consisting of H, a methyl, an allyl, a (E)-propenyl and a phenyl.

In a seventh particular embodiment of the use and method of polysphorin or analogs thereof of formula (E), R7 and R8 together form -O-CH₂-O-, -O-CHF-O-, or - O-CF₂-O-, R9 is H and R12 is selected from the group consisting H, a C₁-C₃ alkyl, a C₂-C₃ alkenyl and a phenyl, more preferably from the group consisting of H, a methyl, an allyl, a (E)-propenyl and a phenyl.

In a eight particular embodiment of the use and method of polysphorin or analogs thereof of formula (E), R10 and R11 are both OMe, OCF₃, OCHF₂, OCH₂F, Me, CF₃, CHF₂, CH₂F or F, more preferably OMe, OCF₃, CF₃ or F, and R12 is selected from the group consisting of H, a C₁-C₃ alkyl, a C₂-C₃ alkenyl and a phenyl, more preferably from the group consisting of H, a methyl, an allyl, a (E)-propenyl and a phenyl.

The conformation between C7 and C8 can be *syn* or *anti.* In a preferred embodiment, the conformation between C7 and C8 is be *syn.*

In a more preferred embodiment of the use and method of polysphorin or analogs thereof of formula (E), R7 and R8 are both OMe or together form -O-CH₂-O-, - O-CF₂-O-, or -O-CHF-O-, R9 is H or OMe, R10 and R11 are OMe, and R12 is selected from the group consisting of H, a C₁-C₆ alkyl, a C₂-C₆ alkenyl and a phenyl optionally substituted by one to three substituents selected in the group consisting of a C₁-C₃ alkyl or alkoxy, halogens, hydroxyl, cyano, amino, and nitro, or any pharmaceutically acceptable salt thereof. Preferably, R12 is selected from the group consisting of H, a C₁-C₃ alkyl, a C₂-C₃ alkenyl and a phenyl, more preferably from the group consisting of H, a methyl, an allyl, a (E)-propenyl and a phenyl.

In a particular embodiment, R7, R8 and R9 are OMe. In another particular embodiment, R7 and R8 are both OMe and R9 is H. In an additional particular embodiment, R7 and R8 together form -O-CH₂-O-, -O-CF₂-O-, or -O-CHF-O-, and R9 is H.

In a first more particularly preferred embodiment of the use and method of polysphorin or analogs thereof of formula (E), R7, R8 and R9 are OMe, R12 is selected from the group consisting of H, a C₁-C₃ alkyl, a C₂-C₃ alkenyl and a phenyl, more preferably from the group consisting of H, a methyl, an allyl, a (E)-propenyl and a phenyl, and R10 and R11 are both OMe.

In a second more particularly preferred embodiment of the use and method of polysphorin or analogs thereof of formula (E), R7 and R8 are both OMe, R9 is H, R12 is selected from the group consisting of H, a C₁-C₃ alkyl, a C₂-C₃ alkenyl and a phenyl, more preferably from the group consisting of H, a methyl, an allyl, a (E)-propenyl and a phenyl, and R10 and R11 are both OMe.

In a third more particularly preferred embodiment of the use and method of polysphorin or analogs thereof of formula (E), R7 and R8 together form -O-CH₂-O-, -O-CF₂-O-, or -O-CHF-O-, R9 is H, R12 is selected from the group consisting of H, a C₁-C₃ alkyl, a C₂-C₃ alkenyl and a phenyl, more preferably from the group consisting of H, a methyl, an allyl, a (E)-propenyl and a phenyl, and R10 and R11 are both OMe.

The polysphorin analogs of formula (E) can be of *syn* or *anti* conformation between C7 and C8, preferably of *syn.* The pharmaceutical composition of the polysphorin or analogs of formula (E) can comprise compound of *syn* or *anti* conformation or a mixture thereof.

The therapeutically effective amount of polysphorin or a polysphorin analog of formula (D) will vary with the particular compound chosen (e.g. depending on the potency, efficacy, and half-life of the compound); the route of administration chosen; the nature of the *Plasmodium* parasite; the phase of the disease (liver or erythrocytic phase); the severity of the infection; the age, size, weight, and physical condition of the patient to be treated; the medical history of the patient to be treated; the duration of the treatment; the nature of concurrent therapy; the desired therapeutic effect; and like factors, but can nevertheless be routinely determined by the skilled artisan.

Preferably, the medicament is suitable for a dose regimen of from 1 to 15 mg/kg/day of the compound, more preferably of about 7 or 8 mg/kg/day.

Preferably, the medicament comprises a unit dose of from 10 mg to 2 g of the compound, more preferably of from 70 mg to 600 mg.

The pharmaceutical composition comprising the polysphorin or an analog thereof of formula (E) can be formulated as described above. Polysphorin or an analog thereof of formula (E) can be used in combination with other antimalarial drugs or compounds that treat one or several of malaria symptoms. In this case, polysphorin analogs and the other active molecules (such as those disclosed above) can be administered simultaneously or consecutively, by the same route or different routes.

The present invention also provides a new method for preparing polysphorin analogs. The method only comprises four steps from cheap and available starting products, is very efficient and can lead to a *syn* or *anti* selectivity. By this method, new polysphorin analogs with a large diversity of aromatic units Ar¹ and Ar² can be prepared. The method of the present invention for the preparation of a polysphorin analog (i.e., Ar1-CHOH-CHMe-O-Ar2), comprises:
a) substituting the bromide of a first aromatic compound (Arl-Br) by a propylacyl group (-CO-CH₂-CH₃), thereby preparing the corresponding aryl ketone Ar1-CO-CH₂-CH₃;
b) introducing a bromide on the carbon atom in alpha of the carbonyl group in the propylacyl rest, thereby preparing the corresponding bromo aryl ketone Ar1-CO-CHBr-CH₃;
c) substituting the bromide by an ether derivative of a second aromatic compound (Ar²-OH), thereby preparing the corresponding aryl ketone ether Arl-CO-CHMe-OAr2; and
d) reducing the compound obtained from step c), thereby obtaining a polysphorin analog (i.e., Ar1-CHOH-CHMe-O-Ar2),
wherein
Ar1 is an aryl of 6-18 atoms, optionally interrupted by one or several heteroatoms selected from N, O, and S, for instance a phenyl, a diphenyl, a 2-benzimidazolyl, 2-quinolinyl, 1-isoquinolinyl, 2-indolyl or indazolyl, the benzene ring thereof being optionally substituted by one to three substituents selected from the group consisting of a (C₁-C₃)alkyl, preferably a methyl or a fluorinated methyl (i.e., CH₂F, CHF₂ or CF₃), a (C₁-C₃)alkoxy, preferably OMe or a fluorinated methoxy (i.e., OCH₂F, OCHF₂ or OCF₃), a dimethylamino group, a fluoride, or by two adjacent substituents forming together -(CH₂)₃-, -O-CH₂-O-, -O-CF₂-O or -O-CHF-O-;
and Ar2 is a phenyl substituted by three substituents, two substituents in ortho of the main alkoxy group selected from the group consisting of OMe, F, CH₂F, CHF₂ or CF₃, OCH₂F, OCHF₂ and OCF₃, and one substituent in para of the ether bond selected from the group consisting ofH, a C₁-C₆ alkyl, a C₂-C₆ alkenyl and a phenyl, optionally substituted by one to three substituents selected in the group consisting of a bromide, a C₁-C₃ alkyl or alkoxy, halogens, hydroxyl, cyano, amino, and nitro, preferably from the group consisting of H, a methyl, an allyl, a (E)-propenyl and a phenyl.

In a preferred embodiment, Ar1 is selected from the group consisting of the disclosed ones in the following table.

The method can comprise a previous step of providing a bromide derivative of a first aromatic compound (Ar¹-Br).

The step a) is performed in presence of BuLi or *tert*-BuLi at very low temperature (e.g., -78°C) and then *N,N*-dimethylpropionamide is added. Preferably, the reaction is performed in Et₂O. After warming the reaction mixture to room temperature, the mixture is quenched with a NH₄Cl solution. The inventors observed that, when the reaction is performed in Et₂O instead of THF (tetrahydrofuran), there is no lithiation of the carbon in alpha of the difluorobenzodioxol and a good regio-selectivity of the lithiation.

Bromating the carbon in alpha of the carbonyl group during step b) can be performed by several methods well-known by the man skilled in the art. One of these methods is described in Lange et al (J. Med. Chem., 2005, 48, 1823-1838) and uses Br₂ in toluene at room temperature.

Step c) is performed by a nucleophilic substitution well-known by the man skilled in the art. For instance, Forest et al (J. Chem. Soc., Perkin Trans. 1, 1974, 205-209) disclose the nucleophilic substitution by phenolates ArO⁻. The use of a weak base (e.g., K₂CO₃) and an aprotic solvent (e.g., Butan-2-one) allows to avoid elimination side products.

The *syn* or *anti* selectivity is controlled during the diastereoselective reduction of the arylketone during step d) through the choice of the reducing agent (Lee & Lee, Org. Biomol. Chem., 2003, 1, 3957-3966). In particular, a non-chelating reducing agent, for instance Zn(BH₄)₂, leads to an *anti* configuration, whereas a chelating reducing agent, for instance K-selectride^{®} or L-selectride^{®}, leads to a *syn* configuration. In a preferred embodiment, *syn* compounds are obtained by using K-selectride^{®} or L-selectride^{®} dissolved in THF at low temperature. In another preferred embodiment, *anti* compounds are obtained by using Zn(BH₄)₂ in a Et₂O/EtOAc mix.

In a particular embodiment, the method of the present invention for the preparation of a polysphorin analog comprises an additional step e) of functionalisation of the group Ar2 having a bromide substituent in para of the ether bond. The functionalisation can be performed by Suzuki-Miyaura coupling (Miyaura, N. et al. Tetrahedron Lett. 1979, 3437 ; Miyaura, N.; Suzuki, A. Chem. Commun. 1979, 866). In particular, this additional step can be useful for preparing Ar2 having a phenyl in para of the ether bond.

For instance, Ar1 and Ar2 can be selected in the following groups.

| **Ar¹** | **Ar²** |
|---|---|
| 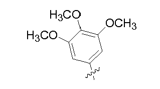 | 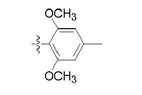 |
| 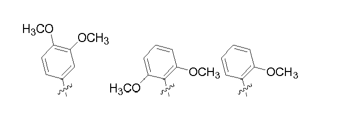 | 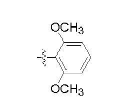 |
| 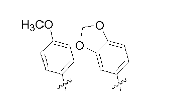 | 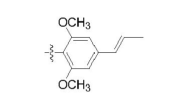 |
| 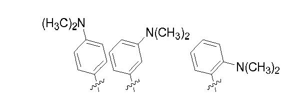 | |
| 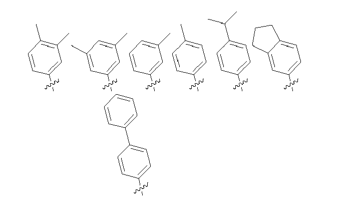 | 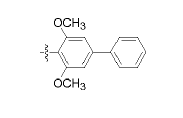 |
| 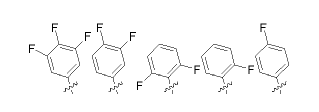 | 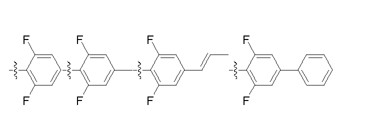 |
| 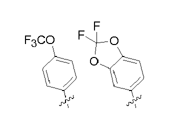 | 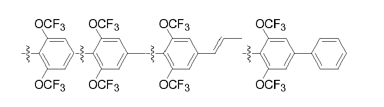 |
| 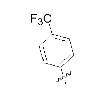 | 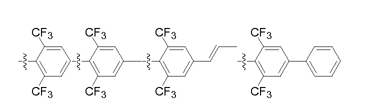 |
| 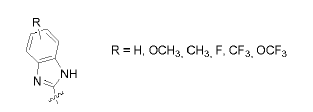 | |
| 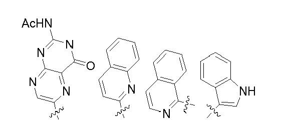 | |

The following examples are given for purposes of illustration and not by way of limitation.

### Examples

### Example 1 : Synthesis of polysphorin analogues.

### 1) Preparation of aryl ketones I

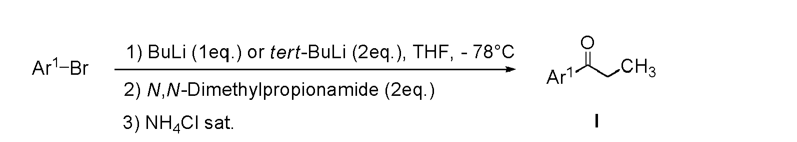

To a solution of arylbromide derivatives (0.05 mol, 1eq.) in Et₂O (100 mL) was added *n*-BuLi (0.05 mol, 32.25 mL of a 1.55M solution in hexanes) or t-BuLi (0.1 mol, 58.8 mL of a 1.7M solution in pentane) dropwise at -78°C under argon atmosphere. The solution was stirred for 1 hour and *N,N*-dimethylpropionamide (11 mL, 0.1 mol, 2eq) was added dropwise. The solution was warmed to room temperature for 1 hour. The mixture was quenched with an aqueous saturated NH₄Cl solution (100 mL) and extracted with Et₂O (4 × 50 mL). The combined organic phases were washed with brine (60 mL) and dried over MgSO₄. Filtration and evaporation of the volatiles *in vacuo* afforded a crude residue, which was purified *via* flash-chromatography on silica gel to give the desired aryl ketone **I.**

Examples of products **I a-c** are described below. They have been synthesized using the procedure described in part 1).

### 1-(3,4,5-trimethoxy-phenyl)-propan-1-one

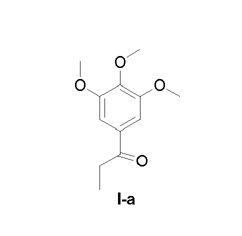

The crude product was purified by chromatography over silica gel (eluent : cyclohexane /ethyl acetate 2 : 1). White solid; R_{f} (cyclohexane/ethyl acetate 2 : 1) = 0.47; Yield : 49%; ¹H NMR (300 MHz, CDCl₃): δ = 7.21 (s, 2 H), 3.91 (s, 6 H), 3.83 (s, 3 H), 2.97 (q, *J* = 6.8 Hz, 2 H), 1.22 (t, *J* = 6.8 Hz, 3 H); ¹³C NMR (75 MHz, CDCl₃): δ = 199.6, 153.0, 142.5, 132.2, 105.4, 60.9, 56.1, 31.5, 8.4.

### 1-(2,2-difluoro-benzo[1,3]dioxol-5-yl)-propan-1-one

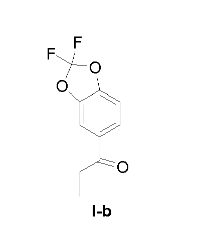

Yellow oil; R_{f} (cyclohexane /ethyl acetate 4 :1) = 0.55; Yield : 62%; ¹H NMR (300 MHz, CDCl₃): δ = 7.79 (d, *J* = 6.9 Hz, 1 H), 7.70 (s, 1 H), 7.13 (d, *J* = 8.4 Hz, 1 H), 2.97 (q, *J* = 6.9 Hz, 2 H), 1.24 (t, *J*=6.9 Hz, 3 H); ¹³C NMR (75 MHz, CDCl₃): δ = 198.2, 146.9, 143.8, 133.5, 131.6 (t, *J* = 255 Hz), 124.6, 109.1, 31.6, 8.0.

### 1-(4-Isopropyl-phenyl)-propan-1-one

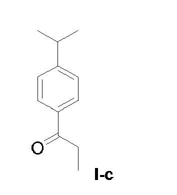

Yellow oil; R_{f} (cyclohexane / ethyl acetate c 10 : 1) = 0.42; Yield : 87%; ¹H NMR (300 MHz, CDCl₃): δ = 7.90 (d, *J* = 8.31 Hz, 2 H), 7.24 (d, *J* = 8.67 Hz, 2 H), 4.13 (q, *J* = 7.32 Hz, 2 H), 2.86 (sept., *J* = 6.96 Hz, 1 H), 1.27 (d, *J* = 6.78 Hz, 6 H), 1.22 (t, *J* = 7.14 Hz, 3 H); ¹³C NMR (75 MHz, CDCl₃): δ = 200.51, 154.30, 134.85, 128.23, 126.61, 34.22, 31.66, 23.67, 8.35.

### 2) Preparation of bromo aryl ketones II

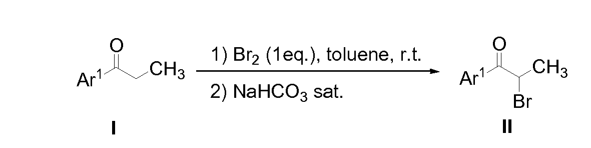

To a solution of aryl ketone **I** (0.02 mol, 1 eq.) in toluene (40 mL) was added dropwise Br₂ (1.03 mL, 0.02 mol, 1 eq) at room temperature. The reaction was stirred for 1 hour and when the reaction was complete as determined by TLC, toluene was removed under reduced pressure and CH₂Cl₂ (100 mL) added to the residue. The solution was washed with an aqueous saturated NaHCO₃ solution (2 × 70 mL). The combined organic phases were dried over MgSO₄, filtered and evaporated *in vacuo* to give a crude residue. The product **II** is pure enough for the further reaction.

Examples of products **II a-c** are described below. They have been synthesised using the procedure described in part 2).

### 2-bromo-1-(3,4,5-trimethoxy-phenyl)-propan-1-one

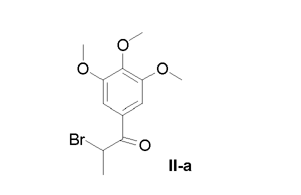

Orange solid; R_{f}(cyclohexane / ethyl acetate 2 : 1) = 0.31; Yield : 98%; m.p. = 104 °C; ¹H NMR (300 MHz, CDCl₃) δ = 7.21 (s, 2 H), 5.18 (q, *J* = 6.6 Hz , 1 H), 3.83 (s, 6 H), 3.81 (s, 3 H), 1.82 (d, *J* = 6.6 Hz, 3 H); ¹³C NMR (75 MHz, CDCl₃): δ =192.2, 153.1, 143.2, 129.1, 106.6 , 61.2, 56.3, 41.2, 20.3.

### 2-bromo-1-(2,2difluoro-benzo[1,3]dioxol-5-yl)-propan-1-one

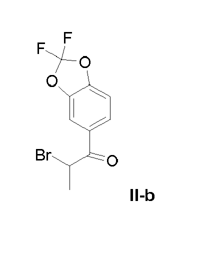

Orange oil; R_{f} (cyclohexane / ethyl acetate 2 : 1) = 0.42; Yield : 70%; ¹H NMR (300 MHz, CDCl₃) δ = 7.87 (d, *J* = 6.6 Hz, 1 H), 7.77 (s, 1H), 7.17 (d, *J* = 6.6 Hz, 1H), 5.20 (q, *J* = 6.6 Hz, 1 H) 1,.92 (d, *J* = 6.6 Hz, 3 H).

### 2-Bromo-1-(4-Isopropyl-phenyl)-propan-1-one

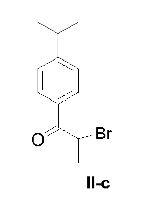

Colorless liquid; R_{f} (cyclohexane/ethyl acetate 10 : 1) = 0.50; Yield : 86%; ¹H NMR (300 MHz, CDCl₃) δ = 7.97 (d, *J* = 8.46 Hz, 2 H), 7.34 (d, *J* = 8.49 Hz, 2 H), 5.29 (q, *J* = 6.78 Hz, 1 H), 2.97 (sept., *J* = 6.96 Hz, 1 H), 1.89 (d, *J* = 6.57 Hz, 3 H), 1.27 (d, *J* = 6.96 Hz, 6 H); ¹³C NMR (75 MHz, CDCl₃): δ =192.6, 155.3, 131.8, 129.2, 127.2, 41.6, 34.3, 23.8, 20.2.

### 3) Preparation of aryl keto ether III

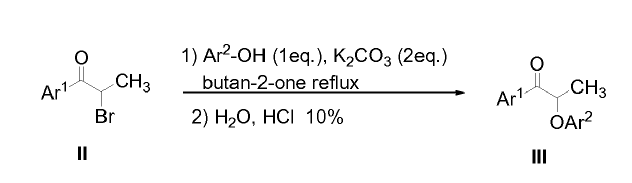

To a solution of aryl phenol (3 mmol, 1 eq) in butan-2-one (20 mL) was added K₂CO₃ (6 mmol, 2 eq) and the bromo aryl ketone **II** (4.5 mmol, 1.5 éq). The mixture was warmed at reflux. After 16 hours stirring, the reaction was quenched with distilled water (100 mL) and acidified with 10% aqueous HCl. The aqueous layer was separated and extracted with ether (4 × 50 mL). The organic layer were combined, washed with water (50 mL), with 1% aqueous NaOH (50 mL) and brine (50 mL). After drying over Na₂SO₄, filtration and concentration *in vacuo* afforded a crude solid, which was crystallized in a mixture of EtOAc/*n*-hexane to give the aryl keto ether **III.**

Examples of products **III a-g** are described below. They have been synthesised using the procedure described in part 3).

### 2-(2,6-dimethoxy-phenoxy)-1-(3,4,5-trimethoxy-phenyl)-propan-1-one

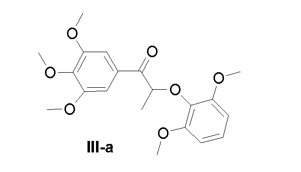

White solid; R_{f} (cyclohexane / ethyl acetate 2 : 1) = 0.36; yield : 75%; m.p. = 95°C; ¹H NMR (300 MHz, CDCl₃): δ = 7.52 (s, 2 H), 7.00 (t, *J* = 8.4 Hz, 1 H), 6.57 (d, *J* = 8.4 Hz, 2 H), 5.30 (q, *J* = 6.6 Hz, 1 H), 3.92 (s, 3 H), 3.89 (s, 6 H), 3.75 (s, 6 H), 1.57 (d, *J* = 6.6 Hz, 3 H); ¹³C NMR (75 MHz, CDCl₃): δ = 197.4, 153.5, 152.8, 142.5, 135.6, 130.5, 123.9, 107.1, 105.2, 80.7, 60.9, 56.2, 55.9, 17.9.

### 2-(2,6-dimethoxy-4-methyl-phenoxy)-1-(3,4,5-trimethoxy-phenyl)-propan-1-one

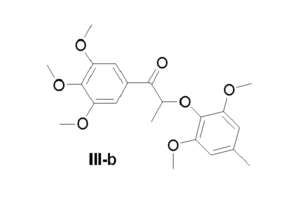

Yellow pale solid; R_{f} (cyclohexane / ethyl acetate 2 : 1) = 0.43; yield : 83%; m.p. : 97-98 °C; ¹H NMR (300 MHz, CDCl₃) δ = : 7.52 (s, 2 H), 6.37 (s, 2 H), 5.26 (q, *J* = 6.6 Hz, 1 H), 3.93 (s, 3 H), 3.89 (s, 6 H), 3.74 (s, 6 H), 2.31 (s, 3 H), 1.56 (d, *J* = 6.6 Hz, 3 H); ¹³C NMR (75 MHz, CDCl₃): δ = 197.6, 153.0, 152.8, 142.4, 133.9, 133.2, 130.5, 107.1, 105.9, 80.7, 60.9, 56.2, 55.8, 21.8, 17.8.

### 2-(4-bromo-2,6-dimethoxy-phenoxy)-1-(3,4,5-trimethoxy-phenyl)-propan-1-one

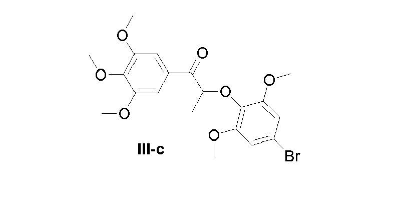

White solid; R_{f} (cyclohexane/ ethyl acetate 2 : 1) = 0.47; Yield : 85%; m.p. : 117°C; ¹H NMR (300 MHz, CDCl₃): δ = 7.52 (s, 2 H), 6.71 (s, 2 H), 5.27 (q, *J* = 6.6 Hz, 1 H), 3.93 (s, 3 H), 3.90 (s, 6 H), 3.74 (s, 6 H) , 1.56 (d, *J* = 6.6 Hz, 3 H). ¹³C NMR (75 MHz, CDCl₃): δ = 197.0, 153.8, 152.8, 142.6, 134.8, 130.2, 116.4, 108.9, 107.0, 80.7, 60.9, 56.2, 56.1, 17.9.

### 2-(4-bromo-2,6-fluoro-phenoxy)-1-(3,4,5-trimethoxy-phenyl)-propan-1-one

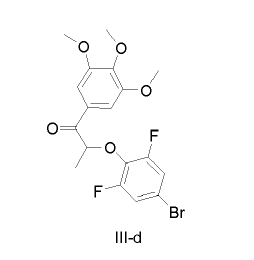

White solid; R_{f} (cyclohexane / ethyl acetate 2 :1 ) = 0.50 ; Yield : 67%; m.p. = 112-113°C; ¹ NMR H (300 MHz, CDCl₃): δ = 7.34 (s, 2 H), 7.10 (d, *J* = 7.8 Hz, 2 H) 5.57 (q, *J* = 6.6 Hz, 1 H), 3.94 (s, 3 H), 3.92 (s, 6 H), 1.65 (d, *J* = 6.6 Hz, 3 H); ¹³C NMR (75 MHz, CDCl₃): δ = 195.3, 155.7, 153.1, 143.2, 133.5 133.8, 129.1, 116.1, 114.5, 106.6, 80.7, 60.9, 56.2, 18.4.

### 2-(3-Trifluoromethyl-phenoxy)-1-(3,4,5-trimethoxy-phenyl)-propan-1-one

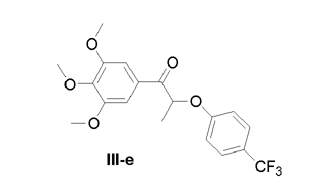

Yellow solid; R_{f} (cyclohexane / ethyl acetate 2 : 1) = 0.46; Yield : 90%; ¹ H NMR (300 MHz, CDCl₃): δ = 7.42 (d, *J* = 8.46 Hz, 2 H), 7.17 (s, 2 H), 6.84 (d, *J* = 8.49 Hz, 2H), 5.36 (q, *J*=6.78 Hz, 1 H), 3.84 (s, 3 H), 3.80 (s, 6 H), 1.67 (d, *J* = 6.78 Hz, 3 H) ; ¹³C NMR (75 MHz, CDCl₃) δ = 197.6, 162.1, 148.5, 137.6, 131.7, 125.9, 122.6, 119.3, 114.5, 107.5, 86.5, 56.6, 56.3, 14.5.

### 2-(3-(Methyl)-1,5-dimethoxy)-1-(3-Isopropyl-phenyl)-propan-1-one

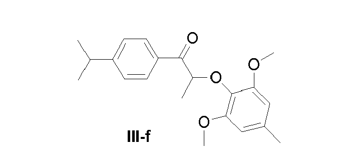

Colorless liquid ; R_{f} (cyclohexane / ethyl acetate 2 : 1) = 0.51; Yield : 86% ;¹ H NMR (300 MHz, CDCl₃): δ = 8.10 (d, *J* = 8.46 Hz, 2 H), 7.29 (d, *J* = 8.28 Hz, 2 H), 5.26 (q, *J* = 6.78 Hz, 2 H), 3.71 (s, 6 H), 2.94 (sept., *J* = 8.28 Hz, 1 H), 2.29 (s, 3 H), 1.56 (d, *J* = 6.78 Hz, 3 H,), 1.26 (d, *J* = 6.78 Hz, 6 H).

### 1-(2,2-difluroro-benzo[1,3]dioxol-5-yl)-2-(2,6-dimethoxy-phenoxy)-propan-1-one

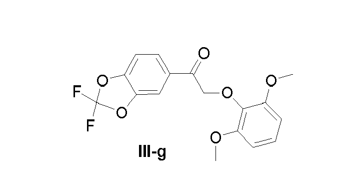

White solid; R_{f}(cyclohexane/ethyl acetate 2 : 1) = 0.58; Yield : 90%; m.p. : 102-103 °C; ¹ H NMR (300 MHz, CDCl₃) : δ = 8.15 (s, 1 H), 8.10 (d, *J* = 8.5 Hz, 1 H), 7.12 (d, *J* = 8.1 Hz, 1 H), 7.01 (t, *J* = 8.1 Hz, 1 H), 6.58 (d, *J* = 8.1 Hz, 2 H), 5.17 (q, *J* = 6.6 Hz, 1 H), 3.77 (s, 6 H), 1.55 (d, *J* = 8.1 Hz, 3 H); ¹³C NMR (75 MHz, CDCl₃): δ = 196.7, 153.4, 146.8, 143.7, 135.4, 131.7 (t, *J* = 253 Hz), 131.6, 126.8, 124.2, 111.1, 108.9, 105.2,81.9,55.8, 17.9.

### 1-(2,2-difluroro-benzo[1,3]dioxol-5-yl)-2-(2,6-dimethoxy-4-methyl-phenoxy)-propan-1-one

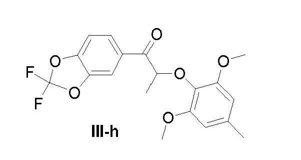

White solid; Yield : 77%; R_{f} (cyclohexane/ethyl acetate 2 : 1) = 0.61; m.p. = 104.5 °C; ¹ H NMR (300 MHz, CDCl₃): δ = 8.15 (s, 1 H), 8.10 (d, *J* = 8.5 Hz, 1 H), 7.12 (d, *J* = 8.5 Hz, 1 H), 6.38 (s, 2 H), 5.12 (q, *J* = 6.6 Hz, 1 H), 3.75 (s, 6 H), 2.31 (s, 3 H), 1.55 (d, *J*= 6.9 Hz, 3 H); ¹³C NMR (100 MHz, CDCl₃): δ = 197.2, 153.3, 147.2, 144.1, 134.6, 133.3, 132.0 (t, *J*= 250 Hz), 127.2, 111.6, 109.3, 106.3 (2 C), 82.3, 56.1 (2 C), 22.3, 18.2.

### 1-(2,2-difluroro-benzo[1,3]dioxol-5-yl)-2-(4-bromo-2,6-dimethoxy-phenoxy)-propan-1-one

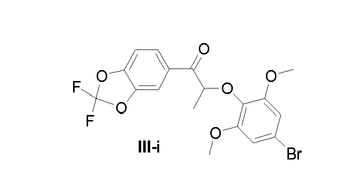

White solid; R_{f} (cyclohexane / ethyl acetate 2 : 1) = 0.60; Yield : 84%; m.p. = 117.5 °C; ¹ H NMR (300 MHz, CDCl₃): δ = 8.09 (s, 1 H), 8.06 (d, *J* = 7.8 Hz, 1 H), 7.13 (d, *J*= 7.8 Hz, 1 H), 6.71 (s, 2 H), 5.15 (q, *J*= 6.9 Hz, 1 H), 3.76 (s, 6 H), 1.54 (d, *J* = 6.9 Hz, 3 H); ¹³C NMR (75 MHz, CDCl₃): δ = 196.2, 153.7, 146.9, 143.7, 134.5, 131.6 (t, *J*= 255 Hz), 131.3, 126.7, 116.7 , 110.7, 109.0, 81.8, 56.0, 17.8.

### 1-(2,2-difluroro-benzo[1,3]dioxol-5-yl)-2-(4-bromo-2,6-difluoro-phenoxy)-propan-1-one

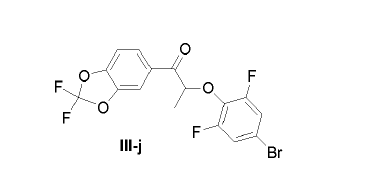

White solid; R_{f} (cyclohexane / ethyl acetate 2 : 1) = 0.7; Yield : 59%; m.p. = 85 °C; ¹ H NMR (300 MHz, CDCl₃): δ = 7.92 (dd, *J₁* = 8.4 Hz, *J₂* = 1.5 Hz , 1H), 7.81 (d, *J*= 1.5Hz, 1H), 7.16 (d, *J*= 8.2Hz, 1H), 7.07 (s, 2 H), 5.48 (q, *J*= 6.6 Hz, 1H), 1.65 (d, *J*= 6.6 Hz, 3 H); ¹³C NMR (100 MHz, CDCl₃): δ = 195.0, 156.0 (d, *J*= 250 Hz), 147.8, 144.6, 133.6, 132.0 (t, *J* = 250 Hz), 131.0, 126.7, 116.6 (d, J = 25 Hz), 115.2, 110.6, 109.8, 81.7, 18.8.

### 4) Stereolective reduction of III affording syn-IV

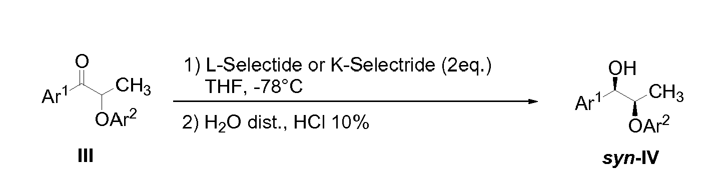

To a solution of aryl keto ether **III** (0.27 mmol, 1eq) in THF (6 mL) L- or K-Selectride (0.54 mmol, 0.54 mL of a 1M solution of THF) was added dropwise at -78°C under argon atmosphere. The reaction was monitored by TLC and after complete conversion of the ketone, the mixture was warmed to room temperature. The reaction was poured in distilled water (100 mL), acidified with a solution of HCl 10%, extracted with CH₂Cl₂ (4 × 50 mL), washed with brine (50 mL) and dried over Na₂SO₄. After concentration *in vacuo,* the crude residue was purified by flash chromatography on silica gel to give a colorless oil.

Examples of products **IV a-e syn** are described below. They have been synthesised using the procedure described in part 4).

### 2-(2,6-dimethoxy-phenoxy)-1-(3,4,5-trimethoxy-phenyl)-propan-1-ol

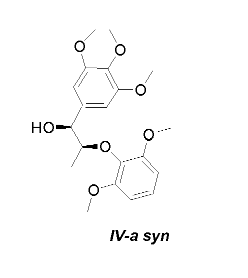

The crude product was purified by flash chromatography over silica gel (eluent cyclohexane/EtOAc 2 : 1). Colorless oil ; R_{f} (cyclohexane / ethyl acetate 1 : 1) = 0.39; yield : 92%; selectivity *anti* / *syn :* 0 :100. ¹ H NMR (300 MHz, CDCl₃): δ = 7.03 (t, *J* = 8.1 Hz, 1 H), 6.62 (d, *J* = 8.1 Hz, 2 H), 6.59 (s, 2 H), 4.94 (s, 1 H, OH), 4.61 (d, *J* = 8.1 Hz, 1 H), 4.01 (quint, *J* = 6.6 Hz, 1 H), 3.89 (s, 6 H), 3.85 (s, 6 H), 3. 81 (s, 3 H), 1.20 (d, *J* = 6.6 Hz, 3 H); ¹³C NMR (75 MHz, CDCl₃): δ = 153.2, 137.1, 136.5, 136.0, 124.1, 105.4, 104.4, 86.2, 79.8, 60.9, 56.1, 18.6.

### 2-(2,6-dimethoxy-4-methyl-phenoxy)-1-(3,4,5-trimethoxy-phenyl)-propan-1-ol

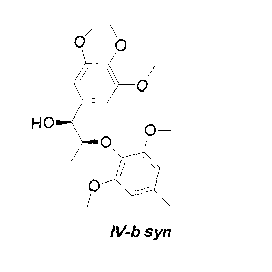

Colorless oil; R_{f} (cyclohexane / ethyl acetate 1 : 1) = 0.46; Yield :95% ; Selectivity *anti l syn* : 0 :100. ¹ H NMR (300 MHz, CDCl₃): δ = 6.58 (s, 2 H), 6.43 (s , 2 H), 4.59 (d, *J*= 8.4 Hz, 1 H), 3.93 (qd, *J₁* = 6.0 Hz, *J₂* = 1.5 Hz, 1H), 3.86 (6 H, s), 3.84 (s, 6 H), 3.81 (s, 3 H), 2.33 (s, 3 H), 1.21 (d, *J*= 6.0 Hz, 3 H); ¹³C NMR (75 MHz, CDCl₃): δ = 153.1, 152.5, 137.6, 136.5, 134.8, 133.7, 106.1, 104.4, 86.3, 79.4, 60.8, 56.1, 56.0, 21.8, 17.7.

### 2-(4-bromo-2,6-dimethoxy-phenoxy)-1-(3,4,5-trimethoxy-phenyl)-propan-1-ol

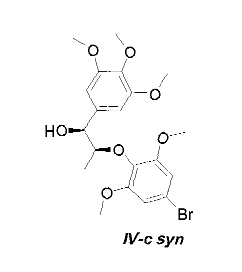

Colorless oil ; R_{f} (cyclohexane / ethyl acetate 1:1 ) = 0.59 ; Yield : 89%; Selectivity *anti l syn* : 0:100. ¹ NMR H (300 MHz, CDCl₃): δ = 6.76 (s, 2 H), 6.58 (s , 2 H), 4.64 (d, *J* = 1.8 Hz, 1 H, OH), 4.58 (dd, *J₁* = 8.1 Hz, *J₂*= 1.8 Hz, 1 H), 4.01 (qd, *J₁* = 6.6 Hz, *J₂* = 6.3 Hz, 1 H), 3.87 (s, 6 H), 3.85 (s, 6 H), 3.82 (s, 3 H), 1.20 (d, *J* = 6.3 Hz, 3 H); ¹³C NMR (75 MHz, CDCl₃): δ = 153.4, 153.1, 137.7, 136.2 116.2, 109.1, 104.3, 86.2, 79.1, 60.8, 56.1, 17.6.

### 2-(4-bromo-2,6-fluoro-phenoxy)-1-(3,4,5-trimethoxy-phenyl)-propan-1-of

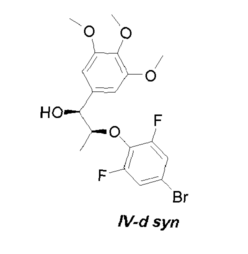

Colorless oil ; R_{f} (cyclohexane / ethyl acetate 1 :1 ) = 0.65 ; Yield : 88%; Selectivity *anti* / *syn :* 0 :100. ¹ NMR H (300 MHz, CDCl₃): δ = 7.11 (d, *J* = 7.8 Hz, 2 H), 6.62 (s, 2 H), 4.68 (d, *J* = 7.5 Hz, 1 H), 4.3 (quint., *J* = 6.6 Hz, 1 H), 3.87 (s, 6 H), 3.83 (s, 3 H), 1.14 (d, *J*= 6.6 Hz, 3 H); ¹³C NMR (75 MHz, CDCl₃): δ = 156.1, 153.3, 137.9, 135.1 133.8, 116.1, 114.6, 104.0, 85.2, 78.4, 60.8, 56.2, 14.1.

### 2-(4-trifluoromethyl-phenoxy)-1-(3,4,5-trimethoxy-phenyl)-propan-1-ol

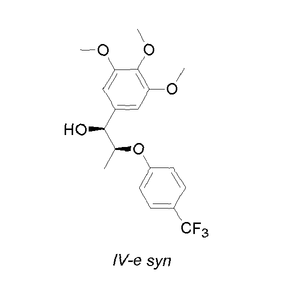

Colorless oil; R_{f} (cyclohexane / ethyl acetate 1 : 1) = 0.32; Yield 90%; ¹ H NMR (300 MHz, CDCl₃): δ = 7.56 (d, *J* = 8.46 Hz, 2 H), 7.02 (d, *J*= 8.46 Hz, 2 H), 6.64 (s, 2 H), 4.66 (d, *J* = 7.35 Hz, 1 H), 4.49 (quint., *J* = 6.03 Hz, 1 H), 3.86-3.90 (s + m, 7 H), 3.84 (s, 3 H), 1.17 (d, *J*= 3.57 Hz, 3 H); ¹³C NMR (75 MHz, CDCl₃): δ =162.1, 153.4, 135.1, 127.1, 122.6, 119.3, 115.9, 104.3, 78.9, 78.0, 60.8, 56.2, 15.8.

### 1-(2,2-difluroro-benzo[1,3]dioxol-5-yl)-2-(2,6-dimethoxy-phenoxy)-propan-1-ol

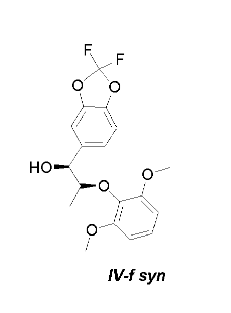

Colorless oil; R_{f} (cyclohexane / ethyl acetate 2 : 1) = 0.54; Yield : 67%; Selectivity *anti* / *syn* : 0 :100; ¹ H NMR (300 MHz, CDCl₃): δ = 7.13 (d, *J* = 1.5 Hz, 1 H), 7.09 (dd, *J₁* = 8.1 Hz, *J₂* = 1.5 Hz, 1 H), 7.06 (d, *J* = 8.1 Hz, 1 H), 7.01 (t, *J* = 8.4 Hz, 1 H), 6.63 (d, *J* = 8.4 Hz, 2 H), 4.95 (s, 1 H, OH), 4.68 (d, *J* = 8.1 Hz, 1 H), 3.98 (quint., *J*= 6.3 Hz, 1 H), 3.89 (s, 6 H) , 1.20 (d, *J*= 6.3 Hz, 3 H); ¹³C NMR (75 MHz, CDCl₃) δ = 153.0, 143.8, 143.2, 137.3, 136.6, 131.6 (t, *J*= 253 Hz), 123.9, 122.8, 108.9, 108.4, 105.3, 85.8, 78.6, 56.0, 17.4.

### 1-(2,2-difluroro-benzo[1,3]dioxol-5-yl)-2-(2,6-dimethoxy-4-methyl-phenoxy)-propan-1-ol

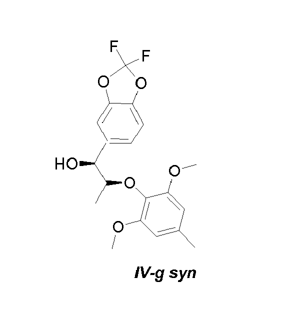

Colorless oil; R_{f} (cyclohexane / ethyl acetate 2 : 1) = 0.55; Yield : 80%; Selectivity *anti* / *syn* : 0 :100. ¹ H NMR (300 MHz, CDCl₃) : δ = 7.13 (d, *J*= 1.5 Hz, 1 H), 7.08 (dd, *J₁* = 8.1 Hz, *J₂* = 1.5 Hz, 1 H), 6.99 (d, *J*= 8.1 Hz, 1 H), 6.42 (s, 2 H), 4.98 (s, 1 H, OH), 4.67 (d, *J*= 8.1 Hz, 1 H), 3.92 (quint, *J*= 8.1 Hz, 1 H), 3.87 (s, 6 H), 2.34 (s, 3 H), 1.18 (d, *J*= 6.6 Hz, 3 H). ¹³C NMR (75 MHz, CDCl₃): δ =152.5, 143.8, 143.2, 137.4, 134.3, 133.9, 131.6 (t, *J*= 255 Hz), 122.8, 108.9, 108.5, 106.0, 85.9, 78.6, 55.9, 21.8, 17.4.

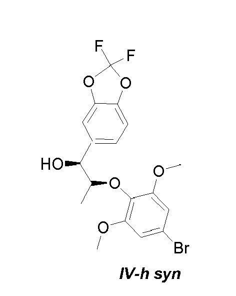

Colorless oil; R_{f} (cyclohexane / ethyl acetate 2 : 1) = 0.69; Yield : 80%; Selectivity *anti* / *syn* : 0 :100. ¹ H NMR (300 MHz, CDCl₃) : δ = 7.13 (d, *J*= 1.5 Hz, 1 H), 7.08 (dd, *J₁* = 8.4 Hz, *J₂* = 1.5 Hz, 1 H), 7.00 (d, *J* = 8.4 Hz, 1 H), 6.76 (s, 2 H), 4.67 (d, *J* = 7.8 Hz, 1 H), 3.97 (q, *J* = 6.3 Hz, 1 H), 3.87 (s, 6 H), 1.17 (d, *J* = 6.3 Hz, 3 H); ¹³C NMR (75 MHz, CDCl₃): δ 153.4, 143.8, 143.3, 137.0, 135.7, 131.6 (t, *J*= 253 Hz), 122.6, 116.4, 109.1, 108.9, 108.4, 85.6, 78.3, 56.2, 17.4.

### 5) Stereoselective reduction of III affording anti-IV

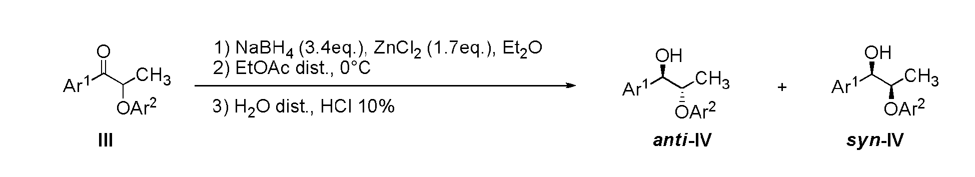

ZnCl₂ previously flame dried (0.45 mmol, 1.7 eq), NaBH₄ (0.9 mmol, 3.4 eq) were suspended in Et₂O (3 mL) under argon atmosphere. The solution was stirred during 3h, in order to prepare Zn(BH₄)₂. The mixture was transferred *via* canula in a second flask containing a solution of aryl ketone in ethyl acetate at 0°C. The reaction was stirred for two hours and poured in distilled water (100 mL), acidified with a 10% aqueous solution of HCl, extracted with Et₂O (4 × 50 mL), washed with brine (50 mL) and dried over Na₂SO₄. Filtration and concentration *in vacuo* afforded a crude residue containing a mixture *syn* and *anti* product. The *anti* product was obtained by flash chromatography on silica gel.

Examples of products **IV anti a-g** are described below. They have been synthesised using the procedure described in part 5).

### 2-(2,6-dimethoxy-phenoxy)-1-(3,4,5-trimethoxy-phenyl)-propan-1-ol

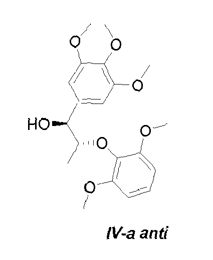

White solid; R_{f} (cyclohexane / ethyl acetate 1 :1) = 0.56; Yield: 99%; Selectivity *anti* / *syn* : 69 : 31. ¹ H NMR (300 MHz, CDCl₃): δ = 7.06 (t, *J*= 8.1 Hz, 1 H), 6.65 (d, *J*= 8.1 Hz, 2 H), 6.55 (s, 2 H), 4.80 (t , *J*= 3 Hz, 1 H), 4.37 (qd, *J₁* = 3 Hz, *J₂* = 6.3 Hz, 1 H), 4.13 (d, *J*= 2.7 Hz, 1 H, OH) 3.90 (s, 6 H), 3.85 (s, 6 H), 3.82 (s, 3 H), 1.14 (d, *J* = 6.3 Hz, 3 H); ¹³C (100 MHz, CDCl₃): δ = 154.3, 153.5, 137.3, 136.0, 135.3, 124.4, 105.7, 103.4, 82.7, 73.6, 61.2, 56.6, 13.2.

### 2-(2,6-dimethoxy-4-methyl-phenoxy)-1-(3,4,5-trimethoxy-phenyl)-propan-1-ol

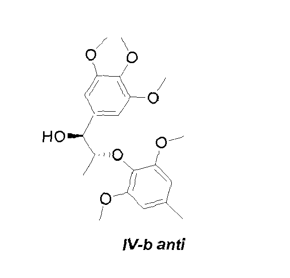

Colorless oil; R_{f} (cyclohexane / ethyl acetate 1 : 1) = 0.45; Yield : 88% ; selectivity *anti* / *syn :* 85 : 15; ¹ H NMR (300 MHz, CDCl₃): δ = 6.54 (s, 2 H), 6.45 (s, 2 H), 4.79 (d, *J* = 2.7 Hz, 1 H), 4.32 (qd, *J₁* = 2.7 Hz, *J₂* = 6.3 Hz, 1 H), 4.13 (s, 1 H, OH), 3.87 (s, 6 H), 3.84 (s, 6 H), 3.82 (s, 3 H), 2.36 (s, 3 H), 1.13 (d, *J*= 6.3 Hz, 3 H); ¹³C NMR (75 MHz, CDCl₃) δ = 153.4, 153.0, 136.8, 135.7, 134.0, 132.4, 106.0, 102.9, 82.2, 73.0, 60.8, 56.1, 21.9, 12.8.

### 2-(4-bromo-2,6-dimethoxy-phenoxy)-1-(3,4,5-trimethoxy-phenyl)-propan-1-ol

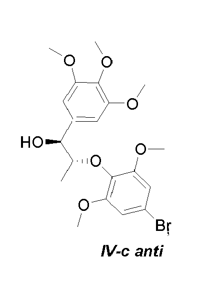

Colorless oil; R_{f} (cyclohexane / ethyl acetate 1 : 1) = 0.62; Yield : 76% ; selectivity *anti* / *syn :* 81 : 19; ¹ H NMR (300 MHz, CDCl₃) : δ = 6.79 (s, 2 H), 6.53 (s, 2 H), 4.78 (d, *J*= 2.7 Hz, 1 H), 4.31 (qd, *J₁* = 2.7 Hz, *J₂* = 6.6 Hz, 1 H), 3.88 (d, 1 H, OH), 3.87 (s, 6 H), 3.85 (s, 6 H), 3.83 (s, 3 H), 1.12 (d, *J*= 6.6 Hz, 3 H); ¹³C NMR (75 MHz, CDCl₃) : δ = 154.2, 153.1, 137.0, 135.3, 134.2, 116.5, 109.1, 103.0, 82.6, 73.3, 60.8, 56.4, 56.1, 12.7.

### 2-(4-trifluoromethyl-phenoxy)-1-(3,4,5-trimethoxy-phenyl)-propan-1-ol

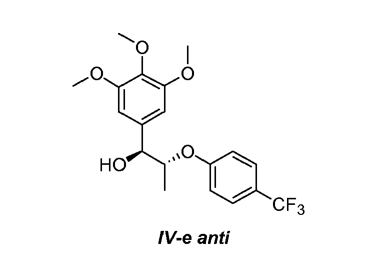

Colorless oil ; R_{f}(cyclohexane / ethyl acetate / CH₂Cl₂, 2 : 1 : 0.5) = 0.2. Yield : 95%. selectivity *anti* / *syn* 80 : 20. Separation with semi-preparative HPLC (Varian Pro Star model 701 with column C-18, 20 cm; eluent H₂O / CH₃CN 1: 1); ¹ H NMR (300 MHz, CDCl₃) : δ = 7.54 (d, *J* = 7.2 Hz, 2 H), 6.97. (d, *J*= 8.7 Hz, 2 H), 6.65 (s, 2 H), 4.68 (s, 1 H), 4.44-4.85 (m, 2 H), 3.86 (s, 6 H), 3.85 (s, 3 H), 1.10 (d, *J* = 6.0 Hz, 3 H); ¹³C NMR (75 MHz, CDCl₃): δ =162.1, 153.4, 135.1, 127.1, 122.6, 119.3, 115.9, 104.3, 78.9, 78.0, 60.8, 56.2, 15.8.

### 1-(2,2-difluroro-benzo[1,3]dioxol-5-yl)-2-(2,6-dimethoxy-phenoxy)-propan-1-ol

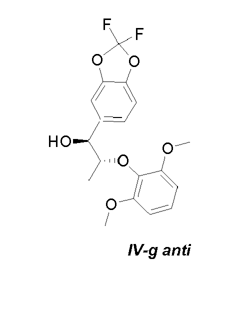

Colorless oil; R_{f} (cyclohexane / ethyl acetate 2 : 1) = 0.56; Yield : 85%; Selectivity *anti* / *syn :* 82 :18; ¹ H NMR (300 MHz, CDCl₃) : δ = 7.14 (s, 1 H), 7.06 (t, *J* = 8.1 Hz, 1 H), 6.98 (s, 2 H), 6.65 (d, *J*= 8.1 Hz, 2 H), 4.82 (d, *J*= 2.7 Hz, 1 H), 4.38 (qd, *J₁* = 6.6 Hz, *J₂* = 2.7 Hz, 1 H), 4.20 (s, 1 H, OH), 3.90 (s, 6 H), 1.11 (d, *J* = 6.6 Hz, 3 H) ; ¹³C NMR (75 MHz, CDCl₃) δ = 153.8, 143.8, 142.6, 136.5, 135.0, 131.6 (t, *J* = 253 Hz), 124.1, 121.0, 108.8, 107.7, 105.4, 82.0, 72.8, 56.1, 12.8.

### 1-(2,2-difluroro-benzo[1,3]dioxol-5-yl)-2-(4-bromo-2,6-dimethoxy-phenoxy)-propan-1-ol

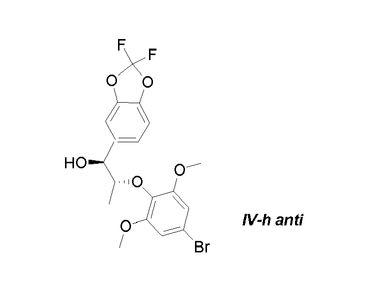

Colorless oil; R_{f} (cyclohexane / ethyl acetate 2 :1 ) = 0.7 ; yield: 84% ; selectivity *anti* / *syn :* 76 : 26. ¹ H NMR (300 MHz, CDCl₃) : δ = 7.13 (s, 1 H), 6.98 (s, 2 H), 6.78 (s, 2 H), 4.81 (t, J = 2.7 Hz, 1 H), 4.32 (qd, *J₁* = 2.7 Hz, *J₂* = 6.3 Hz, 1 H), 3.95 (d, J= 2.7 Hz, 1 H, OH), 3.88 (s, 6 H), 1.09 (d, J= 6.3 Hz, 3 H); ¹³C NMR (75 MHz, CDCl₃): δ =154.1, 143.9, 142.7, 136.2, 134.0, 131.7 (t, J = 255 Hz), 121.1, 116.7, 109.1, 108.9, 107.6, 82.3, 72.9, 56.4, 12.7.

### 1-(2,2-difluroro-benzo[1,3]dioxol-5-yl)-2-(4-methyl-2,6-dimethoxy-phenoxy)-propan-1-ol

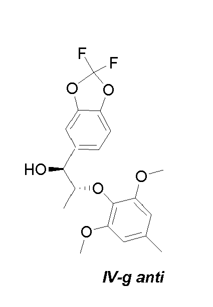

Colorless oil; R_{f} (cyclohexane / ethyl acetate 2 : 1) = 0.49; Yield :90% ; selectivity *anti* / *syn :* 68 : 32; ¹ H NMR (400 MHz, CDCl₃) : δ = 7.14 (s, 1 H), 6.98 (s, 2 H) , 6.45 (s , 2 H), 4.82 (t, *J* = 2 Hz, 1 H), 4.33 (qd, *J₁* = 2 Hz, *J₂* = 6.8 Hz, 1 H), 4.21 (d, *J*= 2 Hz, 1 H, OH), 3.88 (s, 6 H), 2.36 (s, 3 H), 1.11 (d, *J*= 6.8 Hz, 3 H); ¹³C NMR (75 MHz, CDCl₃): δ = 153.6, 144.2, 143.0, 136.9, 134.6, 132.7, 132.0 (t, *J* = 253 Hz), 121.4, 109.2, 108.0, 106.5, 82.3, 73.1, 56.5, 22.3, 13.2.

### 6) Suzuki-Miyaura cross-coupling of IV affording to a new analog IVa

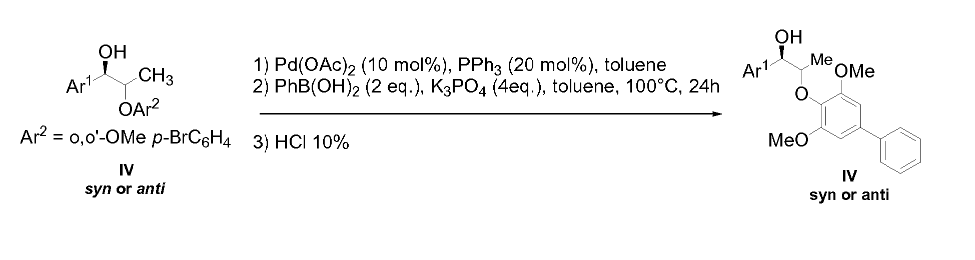

To a solution of a polysphorin analogue **IV** (0.22 mmol, 1 eq) in toluene (1 mL), Pd(OAc)₂ (0.022 mmol, 10 mol%) and triphenylphosphine (0.044 mmol, 20 mol%) were added and stirred 30 min at room temperature under argon atmosphere. The mixture was transferred to a solution of phenylboronic acid (0.44 mmol, 2 eq.) and K₃PO₄ (0.88 mmol, 4 eq.) in toluene (1 mL). After 24 hours heating at 100°C and stirring, the reaction was cooled to room temperature and quenched with 10% aqueous HCl. The reaction was extracted with AcOEt (3 × 25 mL), washed with brine (50 mL) and dried over Na₂SO₄. After concentration *in vacuo,* the crude residue was purified by flash chromatography on silica gel to give a colorless oil.

### 2-(3,5-dimethoxy-biphenyl-4-yloxy)-1-(3,4,5-trimethoxy-phenyl)-propan-1-ol

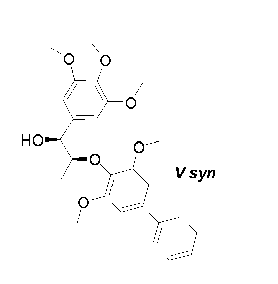

Colorless oil ; R_{f} (cyclohexane / ethyl acetate 1 1 ) = 0.41 ; Yield : 74%; ¹ NMR H (300 MHz, CDCl₃) : δ = 7.58 (dd, *J₁* = 7.2 Hz, *J2* = 1.5 Hz, 2 H), 7.45 (td, *J₁* = 7.2 Hz, *J₂* = 1.5 Hz, 2 H), 7.35 (td, *J₁* = 6.6 Hz, *J2* = 1.5 Hz, 2 H), 6.83 (s, 2 H) 6.62 (s, 2 H), 4.93 (s, 1 H), 4.66 (d, *J* = 7.8 Hz 1 H), 4.06 (qd, *J₁* = 6.6 Hz, *J₂* = 1.5 Hz, 1 H), 3.96 (s, 6 H), 3.87 (s, 6 H), 3.83 (s, 3 H), 1.27 (d, *J* = 6.6 Hz, 3 H); ¹³C NMR (75 MHz, CDCl₃): δ = 153.4, 152.9, 141.1, 137.6 137.3, 136.5, 136.3, 128.7, 127.4, 127.0, 104.4, 104.3, 86.4, 79.3, 60.8, 56.1, 17.7.

### 2-(3,5-dimethoxy-biphenyl-4-yloxy)-1-(3,4,5-trimethoxy-phenyl)-propan-1-ol

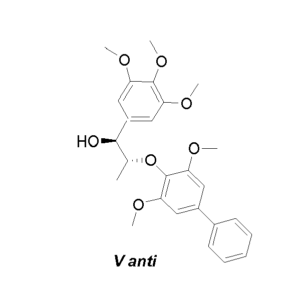

Colorless oil ; R_{f} (cyclohexane / ethyl acetate 1 :1) = 0.41 ; Yield : 74%; ¹ NMR H (300 MHz, CDCl₃) : δ = 7.60 (dd, *J₁* = 7.2 Hz, *J2* = 1.5 Hz, 2 H), 7.46 (td, *J₁* = 7.2 Hz, *J₂* = 1.5 Hz, 2 H), 7.37 (td, *J₁* = 6.6 Hz, *J₂* = 1.5 Hz, 2 H), 6.85 (s, 2 H) 6.58 (s, 2 H), 4.86 (t, *J* = 3 Hz, 1H), 4.43 (qd, *J₁* = 6.6 Hz, *J₂* = 3 Hz, 1 H), 4.12 (d, *J* = 2.4 Hz, 1 H), 3.96 (s, 6 H), 3.86 (s, 6 H), 3.83 (s, 3 H), 1.18 (d, *J* = 6.6 Hz, 3 H). ¹³C NMR (75 MHz, CDCl₃): δ = 153.8, 153.1, 141.0, 137.6 136.9, 135.6, 134.3, 128.8, 127.5, 127.0, 104.5, 103.0, 82.5, 73.3, 60.8, 56.2, 12.9.

### 7) Synthesis of different aryl phenols Ar² OH VI (used in part 3))

### 4-bromo 2,6-dimethoxyphenol VI-a

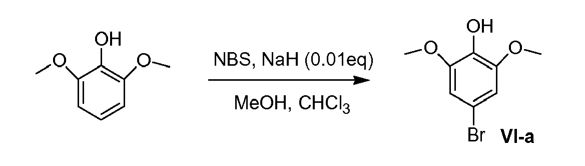

To a solution of 2,6-dimethoxyphenol (25 g, 0.162 mol) in anhydrous CHCl₃ (220 mL) cooled at -45°C was added methanol (1.8 mL) and sodium hydride (62 mg, 162 mmol, 0.01 éq). NBS (30.59 g, 0.172 mol, 1.06 eq) was added and the mixture was stirred at -45°C for 90 minutes. The reaction was monitored by TLC (Cyclohexane/EtOAc 2: 1). After complete conversion of the phenol the mixture was hydrolysed with water (100 mL). The aqueous phase was extracted by CH₂Cl₂ (3×50 mL). The organic layers were combined, washed with saturated aqueous solution of Na₂S₂O₃ (2×100 mL), dried over Na₂SO₄, and evaporated under reduced pressure. A white solid was obtained after recristallization from EtOAc/hexanes. R_{f} (cyclohexane/ethyl acetate 2 :1 ) = 0.53; Yield : 53%; m.p.: 98 °C; NMR ¹ H (300 MHz, CDCl₃) : δ = 6.73 (s, 2 H), 3.90 (s, 1 H), 3.87 (s, 6 H).

### 4-bromo 3,5-difluorobromobenzène VI-b

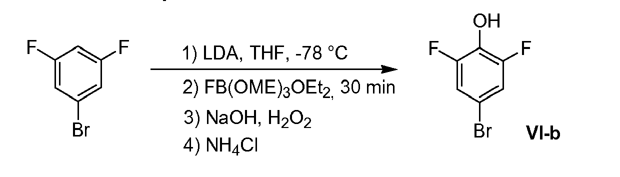

To a solution of diisopropylamine (7.06 mL, 0.05 mol, 1eq), in THF (100 mL) under argon, cooled at -40°C was added butyllithium (1.55M in THF, 31.25 mL, 0.05 mol, 1eq). The mixture was stirred 30 minutes at 0°C and then cooled at -78 °C. To this reaction was added dropwise 3,5 difluorobromobenzene (5.76 mL, 0.05 mol, 1eq). The solution turned on to yellow. The reaction was stirred 2 h at -78°C and FB(OMe)₂.OEt₂ (10.54 mL, 0.05 mol, 1eq) was added dropwise. The mixture was warmed at 0°C and stirred at 0°C for 30 minutes. A solution of aqueous NaOH 3M (19 mL) and H₂O₂ (30% in water, 5.27 mL) was added quickly. The solution was warmed to room temperature during the night (12 hours) and quenched with 10% aqueous HCl. The aqueous layer was extracted by CH₂Cl₂ (4 × 50 mL). The combined organic phases were washed with brine (1 × 70 mL), dried over Na₂SO₄, and evaporated under reduced pressure. The crude product was purified by chromatography over silica gel with cyclohexane/EtOAc 4 : 1. R_{f} (cyclohexane/ ethyl acetate : 4/1) = 0.35; Yield : 29%; Orange oil; NMR ¹H (300 MHz, CDCl₃) : δ = 7.09 (d, *J* = 7.2 Hz, 2 H), 5.08 (s, 1 H, *OH*).

### Preparation of syn and anti polysphorin

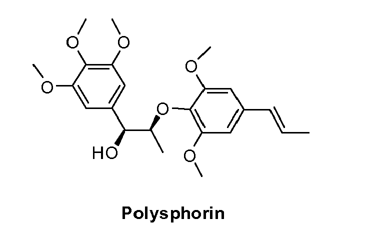

### Preparation of 1-(3,4,5-Trimethoxy-phenyl)-2-propen VIII^{[2]}

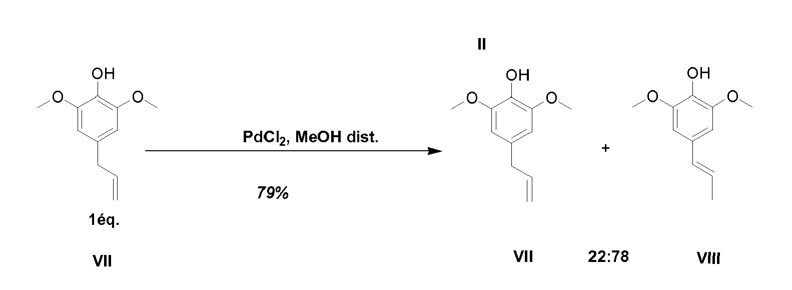

9-Allyl-10-phenylphenanthrene (0,6 g, mmol, leq.) was dissolved in a solution of KOH 20% (15 mL). The mixture was refluxed for 20h. The solvent was evaporated and poured into an aqueous solution of HCl 10%. The solution was extracted with ethyl acetate ( 2 x 50 mL). The organic layer was dried over MgSO₄, filtered and evaporated under reduced pressure. The crude product was purified by chromatography on a silica gel with cyclohexane / ethyl acetate / chloroform: 1 : 0,5 : 1 and cyclohexane / ethyl acetate : 1 :1.

The isolated solution contained a mixture of allylic and vinylic compounds in a 22 : 78 ratio (determined with NMR ¹H) and a yield of 79%.

Orange viscous liquid, R_{f}(cyclohexane : ethyl acetate 1 / 1) = 0.41; ¹H NMR (300 MHz, CDCl₃) mixture of the allylic and vinylic compounds δ = 6.57 (s, 2 H), 6.41 (s, 2 H), 6.3 (s, 1 H); 6.15-5.89 (m, 3H), 5.05-5.12 (m, 2H), 3.88 (s, 6 H,), 3.86 (s, 6 H,), 3.49 (s, 1 H), 3.31 (d, *J* = 6.6 Hz, 2 H), 1.86 (d, *J* = 6.57 Hz, 3 H).

### Preparation of 2-(2,6-dimethoxy-4-propenyl-phenoxy)-1-(3,4,5-trimethoxy-phenyl)-propan-1-one IX and 2-(4-allyl-2,6-dimethoxy-phenoxy)-1-(3,4,5-trimethoxy-phenyl)-propan-1-one X

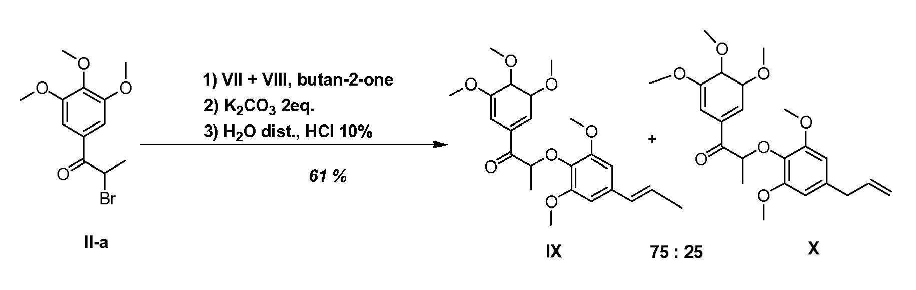

The compound **IX** and X were obtained using the procedure described in part 3).

A mixture of allylic and vinylic compound **IX** and **X** was obtained in a ratio from 25 : 75 respectively. Yield : 61%. The separation was performed by HPLC (Varian Pro Star model 701 with a column C-18 20 cm; eluent water / acetonitrile 4:6).

### Description of compound X:

Viscous liquid; R_{f} (cyclohexane / ethyl acetate 2 : 1) = 0.51; ¹ H NMR (300 MHz, CDCl₃) δ = 6.57 (s, 2 H), 6.41 (s, 2 H), 6.3 (s, 1 H); 6.15-5.89 (m, 3 H), 5.05-5.12 (m, 2 H), 3.88 (s, 6 H,), 3.86 (s, 6 H,), 3.49 (s, 1 H), 3.31 (d, *J* = 6.6 Hz, 2 H), 1.86 (d, *J* = 6.57 Hz, 3 H).

### Description of compound IX:

Viscous liquid; R_{f} (cyclohexane / ethyl acetate 2 : 1) = 0.51. ¹ H NMR (300 MHz, CDCl₃) δ = 7.51 (s, 2 H), 6.54 (s, 2 H), 6.33 (dd, *J* = 15.6 Hz, *J* = 1.5 Hz, 1 H), 6.17 (dq, *J₁* = 15.6 Hz, *J₂* = 6.3 Hz, 1 H), 5.30 (q, *J* = 6.9 Hz, 1 H), 3.92 (s, 3 H), 3.89 (s, 6 H), 3.76 (s, 6 H), 1.88 (dd, *J₁* = 6.3 Hz, *J₂* = 1.5 Hz, 3 H), 1.56 (d, *J* = 6.9 Hz, 3 H). ¹³ C NMR (75 MHz, CDCl₃) δ = 152.81, 136.12, 133.90, 130.75, 125.63, 104.37, 102.93, 86.41, 79.35, 60.79, 56.01, 18.33, 17.68.

### Preparation of the stereoisomer syn from polysphorin XI-syn:

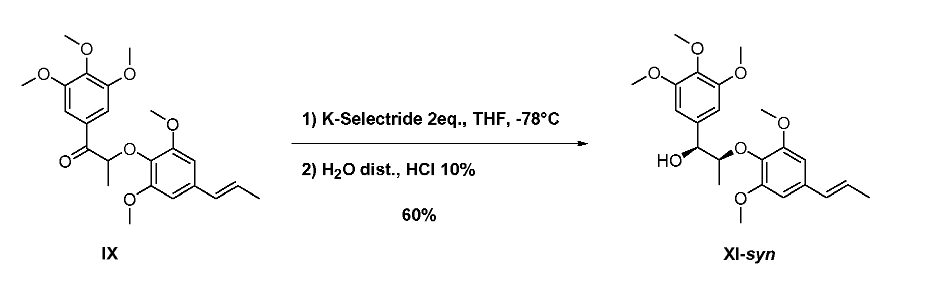

The compound **XI-*syn*** was obtained using the procedure described in part 4).

The crude product was purified by chromatography over silica gel (eluent cyclohexane / EtOAc 2 : 1).

Colorless oil; R_{f} (cyclohexane / ethyl acetate 1 : 1) = 0.31. Yield : 60%; ¹ H NMR (300 MHz, CDCl₃) δ = 6.59 (s, 2 H), 6.58 (s, 2 H), 6.34 (dd, *J₁* = 11.7 Hz, *J₂* = 0.9 Hz, 1 H), 6.17 (dq, *J₁* = 11.7 Hz, *J₂* = 5.1 Hz, 1 H), 4.92 (s, 1 H), 4.5 (d, *J* = 6.3 Hz, 1 H), 4.04-3.94 (m, 1 H), 3.88 (s, 6 H), 3.84 (s, 6 H), 3.81 (s, 3 H), 1.88 (dd, *J₁* = 4.8 Hz, *J₂* = 0.9 Hz, 3 H), 1.22 (d, *J* = 4.8 Hz, 3 H); ¹³ C NMR (75 MHz, CDCl₃) δ = 152.81, 136.12, 133.90, 130.75, 125.63, 104.37, 102.93, 86.41, 79.35, 60.79, 56.01, 18.33, 17.68.

### Preparation of the stereoisomer anti from the polysphorine XI-anti:

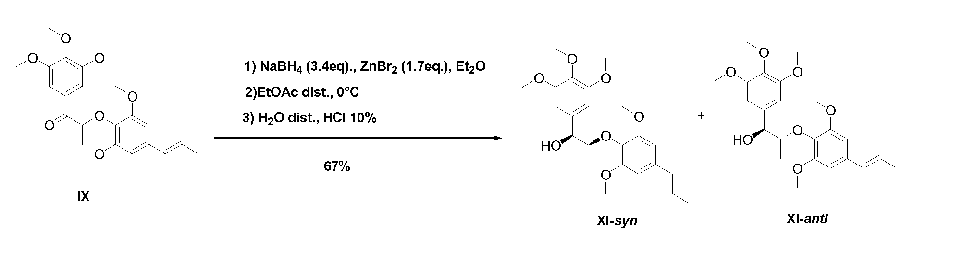

The compound **XI-*anti*** was obtained using the procedure described in part 5).

A first purification was realized by flash chromatography over silica gel (eluent cyclohexane / EtOAc 2 : 1). A second one by HPLC (Varian Pro Star model 701 with a column C-18 20 cm; eluent H₂O / CH₃CN 4 : 6).

Colorless oil, R_{f} (cyclohexane / ethyl acetate 1 : 1) = 0.45; ¹ H NMR (300 MHz, CDCl₃) δ = 6.53 (s, 2 H), 6.45 (s, 2 H), 6.27 (dd, *J₁* = 15.6Hz, *J₂* = 1.5 Hz, 1H), 6.11 (dq, *J₁* = 15.6 Hz, *J₂* = 6.3 Hz, 1H), 4.70 (d, *J* = 2.4 Hz, 1 H), 4.26 (qd, *J* = 7.8 Hz, *J* = 2.7 Hz, 1 H), 4.0-4.04 (m, 1H), 3.81 (s, 6 H), 3.76 (s, 6 H), 3.73 (s, 3 H), 1.90 (dd, *J1 =* 6.6 Hz, J2 = 1.5 Hz, 3 H), 1.05 (d, *J* = 6.3 Hz, 3 H). ¹³ C NMR (75 MHz, CDCl₃) δ = 152.81, 136.12, 133.90, 130.75, 125.63, 104.37, 102.93, 86.41, 79.35, 60.79, 56.01, 18.33, 17.68.

### Example 2: Evaluation of the anti-malarial activity of polysphorin analogs

The activity of polysphorin analogs of the invention has been evaluated at hepatitic and erythrocytic stages of the disease.

### Methods

### Evaluation of the activity against erythrocytic stage

*In vitro* assays were performed using the chloroquine-sensitive (3D7w) clone of *P. falciparum* (David Walliker) which was maintained in continuous culture according to a modified version of the method of Trager and Jensen as described (Trager and Jensen, 1976). The parasitic cultures were incubated at 37°C with 4% CO₂-96% air.

The *in vitro* activities of the drugs were evaluated by using a method previously described (Desjardins et al, 1979) with modifications as follows: briefly, in 96-well plates preloaded with nine concentrations (1.5.10⁻² to 100µM) of all drugs in triplicate, and serial dilutions of chloroquine in positive-control wells, 200 µl of ring stage parasitized erythrocytes (parasitemia, 0.5%; hematocrit, 1.8%) were distributed in each well. After 48 hours, [³H]-hypoxanthine (Amersham) was added to each well and then plates were incubated for 24 hours. Parasites were harvested, and incorporation of radioactivity was determined by liquid scintillation counting (Beckman LS1701, les Ulis, France). The counts-per-minute (cpm) was adjusted relative to controls to obtain percentages of inhibition. The IC₅₀ was estimated by linear regression of log10 of concentration of compounds for data points between plateaus, from the highest concentration in the lower plateau to the lowest concentration in the higher plateau. This estimation was obtained with IC₅₀ estimator software. For the assay, the parasites were synchronized by D-sorbitol treatment.

Toxicity was determined by using the colorimetric 3-(4, 5-dimethylthiazol-2-yl)-2, 5-diphenyltetrazolium bromide (MTT) assay (Mosman., 1983). Viability data were used to plot concentration-effect curves, from which 50% toxic concentrations (TC₅₀) were estimated by linear regression. The selectivity index (SI) was defined as the ratio of the TC₅₀ to the IC₅₀.

### Evaluation of the activity against hepatic stage

Activity against pre-erythrocytic stages was tested by *in vitro* assays with the human parasite *P. falciparum* (NF54 strain) and the rodent parasite *P. yoelii* (265 BY strain). The rodent parasite *P. yoelii* is produced in the insectarium of the INSERM Unit U511 and human parasite *P. falciparum* was obtained from collaboration with R. Sauerwein.

Sporozoites were obtained from infected salivary glands of *Anopheles stephensi* mosquitoes fed on continuous culture of *P. falciparum* (coll R. Sauerwein, Netherlands), or on *P. yoelii* parasitized mice (insectarium of the INSERM Unit U511). After aseptic dissection, salivary glands were homogenized in a glass grinder and diluted in culture medium or sterile phosphate-buffered saline (PBS).

Mice used for these experiments were 7 to 12-week-old female SWISS mice purchased from Janvier (Le Genest saint Isle, France).

### Measurement of IC₅₀ of polysphorin analogs on infected HepG2 cells

A preliminary screening was performed consisting in the infection of human CD81 transfected hepatocarcinome cell line HepG2 (HepG2/CD81) with *P. yoelii.* IC₅₀ (inhibiting concentration 50), TC₅₀ (toxic concentration 50) and TI (therapeutic index, ratio TC₅₀/IC₅₀) were estimated for each drug tested. Selected molecules (small IC₅₀, high TC₅₀ and high TI) were analyzed in human hepatocytes. Briefly, HepG2 cells were plated in 96-well plates for 24 h prior to inoculation with 1x10⁴ of P. *yoelii* sporozoites. Sporozoites-inoculated culture plates were centrifuged for 5 min at 1,500 rpm at 4°C in order to enhance the infection rate as described by Gruner (Gruner et al., 2005). After 3h at 37°C, the cultures were washed and further incubated in fresh medium for 48 h (*P. yoelii*) before quantification of infected cells in triplicate wells.

### Measurement of TC₅₀ of polysphorin analogs on HepG2 cells

Toxicity was determined by using the colorimetric 3-(4,5-dimethylthiazol-2-yl)-2,5-diphenyltetrazolium bromide (MTT) assay (Mosman., 1983). Briefly, HepG2 cells cultures were prepared in 96-well plates as described above. Drugs were tested in triplicate wells at eight concentrations ranging from 6.4 × 10⁻³ µM to 200 µM. One hundred microliters of 0.5 mg/ml MTT (5 mg/ml MTT in water diluted 10x in William's medium without fetal bovine serum) was added to each well, and the plates were incubated for 4 h (37°C, 5% CO₂). The medium was discarded, and the cells were resuspended in ethanol and dimethyl sulfoxide (50/50, vol/vol). The plates were read in an enzyme-linked immunosorbent assay plate reader (550-nm absorbance wavelength and 620-nm reference wavelength). The results were expressed as the percent change in viability compared to drug-free control cultures. Viability data were used to plot concentration-effect curves, from which 50% toxic concentrations (TC₅₀) were estimated by linear regression. Each drug and concentration was tested at least in triplicate. The selectivity index (SI) was defined as the ratio of the TC₅₀ to the IC₅₀.

### Measurement of IC₅₀ of polysphorin analogs on infected hepatocytes

Primary cultures of rodent or human hepatocytes were prepared from liver segments taken from mouse or from adult patients undergoing partial hepatectomy. Hepatocytes were isolated using the two-step enzymatic perfusion technique (Guguen-Guillouzo et al., 1982) and were further purified on a discontinuous Percoll gradient. Then, hepatocytes were seeded in eight-chamber Lab-Tek permanox slides at a density of 1,4x10⁵ cells per cm², and maintained under culture conditions as described in Mahmoudi et al., 2003. After complete adherence to culture plates, rodent and human hepatocytes were infected with *P. yoelii* or *P. falciparum* sporozoites, respectively, aseptically dissected from mosquitoes' salivary glands, as described above. Hepatocytes were inoculated with 6x10⁴ sporozoites per Lab-Tek well. Compounds to be tested were added at the time of infection and/or after penetration of the sporozoites and at different concentrations (three wells per dilution). Every day, medium was replaced by fresh medium containing or not different concentrations of the molecules to be tested. Cultures were arrested at 48 hours (*P. yoelii*) or 6 days (*P. falciparum*) and cells were fixed with methanol. Liver-schizonts were quantified by microscopic examination after fluorescent labeling using a primary mouse antibody directed against *Plasmodium* heat shock protein-70 (Renia et al., 1990) produced in INSERM unit 511 and a secondary goat anti-mouse antibody conjugated with fluorescein isothiocyanate (Sigma Aldrich).

### Measurement of TC₅₀ of polysphorin analogs on hepatocytes

Cytotoxic effect of the compounds was evaluated on human hepatocytes, obtained as described above. The colorimetric MTT assay was used (Mosman., 1983). Briefly, hepatocyte cultures were prepared in 96-well plates as described above. Drugs were tested in triplicate wells at eight concentrations ranging from 6.4 x 10⁻³ µM to 200 µM. One hundred microliters of 0.5 mg/ml MTT (5 mg/ml MTT in water diluted 10x in William's medium without fetal bovine serum) was added to each well, and the plates were incubated for 4 h (37°C, 5% CO₂). The medium was discarded, and the cells were resuspended in ethanol and dimethyl sulfoxide (50/50, vol/vol). The plates were read in an enzyme-linked immunosorbent assay plate reader (550-nm absorbance wavelength and 620-nm reference wavelength). The results were expressed as the percent change in viability compared to drug-free control cultures. Viability data were used to plot concentration-effect curves, from which 50% toxic concentrations (TC₅₀) were estimated by linear regression. Each drug and concentration was tested at least in triplicate. The selectivity index (SI) was defined as the ratio of the TC₅₀ to the IC₅₀.

### Results

The antimalarial activity of compositions comprising polysphorin analogs was evaluated. These compositions comprised either only one diastereoisomere or a mix of *syn* and *anti* diastereoisomers. These compounds were obtained by the method of synthesis of the invention as described above.

Results obtained for these compounds are presented in the table 1 below.

**Table 1: Anti-malarial activity of polysphorin analogs at Hepatic and erythrocytic stages against P. yoelii and P. falciparum.**

| Compounds | Hepatic stage (rodent) *P. yoelii* | | | Hepatic stage (human) *P. Falciparum* | | | Erythrocytic stage *P. falciparum* | | |
|---|---|---|---|---|---|---|---|---|---|
| | IC₅₀ (µM) | TC₅₀ (µM) | SI | IC₅₀ (µM) | TC₅₀ (µM) | SI | IC₅₀ (µM) | TC₅₀ (µM) | SI |
| 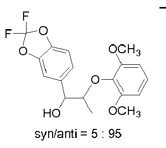 | 20.13 | 54.93 | 2.73 | | | | >40 | 55 | - |
| 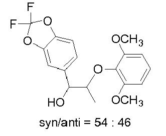 | 20.19 | 53.00 | 2.62 | | | | >40 | 53 | - |
| 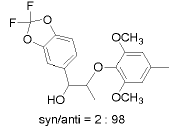 | 19 | 56.83 | 2.99 | | | | 13.6 | 57 | 4.19 |
| 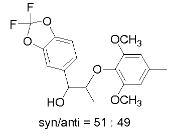 | 5.62 | 53.69 | 9.55 | | | | 13.45 | 53.7 | 3.99 |
| 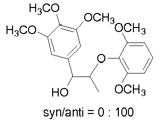 | 28.61 | 57.86 | 2.02 | | | | >40 | 58 | - |
| 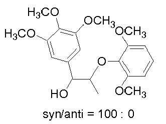 | 2.52 | 0.126 | 0.05 | | | | | | |
| 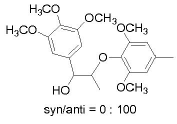 | 50.1 | 47.01 | 0.94 | | | | >40 | 47 | - |
| 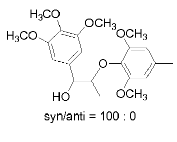 | 0.0045 ±0.005 | 49.54 | 11008 | 0.38 | 124,73 | 328 | >40 | 49.5 | - |
| 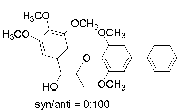 | 3.54 ±4.9 | >50 | >14 | 0.02 | 50 | 2500 | 11.5 | 50 | 4.34 |
| 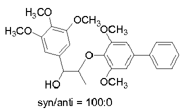 | 3.61 ±4 | >50 | >13.85 | 0.007 | 50 | 7142 | 20 | 50 | 2.5 |
| Primaquine | 0.075 | 12.6 | 168 | | | | | | |
| Chloroquine | | | | | | | 0.04 | | |
| Polysphorin *syn* *anti* | 9.54 9.53 | 96.5 91.7 | 10.11 9.62 | 2.72 3.65 | 70.8 116.8 | 26.02 32 | | | |

### References

Berge, et al. J. Pharmaceutical Sciences, 1977, 66: 1-19
Desjardins et al. Antimicrob Agents Chemother, 1979, 16, 710
Gruner et al. Mol. Biochem. Parasitol, 2005, 142, 184
Guguen-Guillouzo et al. Cell Biol Int Rep, 1982, 6, 625
Mahmoudi et al. Antimicrob Agents Chemother, 2003, 47, 2636
Mosman. J. Immunol. Methods, 1983, 65, 55
Renia et al. Eur J Immunol, 1990, 20, 1445
Tragger and Jensen, Science, 1976, 193,673

## Claims

1. A compound of formula (A) 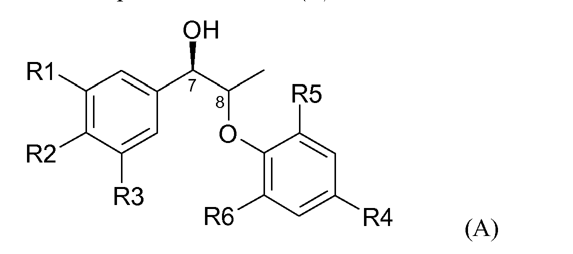 wherein
R1 and R2 are selected from the group consisting of OMe, OCF₃, OCHF₂, OCH₂F, Me, CF₃, CHF₂, CH₂F and F, or together form -O-CH₂-O-, -O-CHF-O-, or -O-CF₂-O-,
R3 is selected from the group consisting of H, OMe, OCF₃, OCHF₂, OCH₂F, Me, CF₃, CHF₂, CH₂F and F,
R5 and R6 are selected from the group consisting of OMe, OCF₃, OCHF₂, OCH₂F, Me, CF₃, CHF₂, CH₂F and F, and
R4 is selected from the group consisting of H, a C₁-C₆ alkyl and a phenyl, optionally substituted by one to three substituents selected in the group consisting of a C₁-C₃ alkyl or alkoxy, halogens, hydroxyl, cyano, amino, and nitro,
or a pharmaceutically acceptable salt thereof.

2. The compound according to claim 1, wherein the compound has the formula (B) : 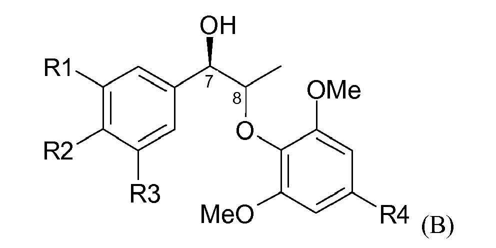 wherein
R1 and R2 are both OMe or together form -O-CH₂-O-, -O-CHF-O-, or -O-CF₂-O-,
R3 is H or OMe, and
R4 is selected from the group consisting of H, a C₁-C₃ alkyl and a phenyl.

3. The compound according to claim 1 or 2, wherein R4 is H, a methyl or a phenyl, preferably a methyl or a phenyl.

4. The compound according to any one of claims 1-3, wherein R1, R2 and R3 are OMe.

5. The compound according to any one of claims 1-3, wherein R1 and R2 together form -O-CH₂-O -O-CF₂-O or -O-CHF-O-, and R3 is H.

6. The compound according to any one of claims 1-5, wherein the conformation between C7 and C8 is *syn,* and R4 is H or a methyl, preferably a methyl.

7. Compound according anyone of claims 1 to 6 as a medicament.

8. Compound according anyone of claims 1 to 6 as an antimalarial prophylactic or therapeutic agent.

9. A pharmaceutical composition comprising a compound according anyone of claims 1 to 6 and optionally a pharmaceutically acceptable carrier and/or excipient.

10. The pharmaceutical composition according to claim 9, further comprising one or several additional antimalarial drugs or compounds that treat one or several of malaria symptoms.

11. Compound according anyone of claims 1 to 6 for preventing or treating malaria, in particular at the hepatic phase.

12. Polysphorin or analog thereof of formula (E) for treating malaria at the hepatic stage, wherein the formula (E) is the following: 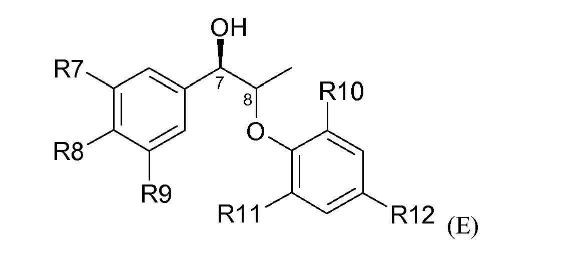 wherein
R7 and R8 are selected from the group consisting of OMe, OCF₃, OCHF₂, OCH₂F, Me, CF₃, CHF₂, CH₂F and F, or together form -O-CH₂-O-, -O-CHF-O-, or -O-CF₂-O-,
R9 is selected from the group consisting of H, OMe, OCF₃, OCHF₂, OCH₂F, Me, CF₃, CHF₂, CH₂F and F,
R10 and R11 are selected from the group consisting of OMe, OCF₃, OCHF₂, OCH₂F, Me, CF₃, CHF₂, CH₂F and F, and
R12 is selected from the group consisting of H, a C₁-C₆ alkyl, a C₂-C₆ alkenyl and a phenyl, optionally substituted by one to three substituents selected in the group consisting of a C₁-C₃ alkyl or alkoxy, halogens, hydroxyl, cyano, amino, and nitro,
or any pharmaceutically acceptable salt thereof.

13. Polysphorin or analog thereof according to claim 12, wherein R12 is selected from the group consisting of H, a C₁-C₃ alkyl, a C₂-C₃ alkenyl and a phenyl, more preferably from the group consisting of H, a methyl, an allyl, a (E)-propenyl and a phenyl.

14. Polysphorin or analog thereof according to claim 12 or 13, wherein R7, R8 and R9 are OMe, or R7 and R8 are both OMe and R9 is H, or R7 and R8 together form -O-CH₂-O-, -O-CF₂-O-, or -O-CHF-O- and R9 is H.

15. Polysphorin or analog thereof according to any one of claim 12-14, wherein R10 and R11 are both OMe.

16. A method for preparing a polysphorin analog (i.e., Ar1-CHOH-CHMe-O-Ar2), comprising:
a) substituting the bromide of a first aromatic compound (Arl-Br) by a propylacyl group (-CO-CH₂-CH₃), thereby preparing the corresponding aryl ketone Ar1-CO-CH₂-CH₃;
b) introducing a bromide on the carbon atom in alpha of the carbonyl group in the propylacyl rest, thereby preparing the corresponding bromo aryl ketone Ar1-CO-CHBr-CH₃;
c) substituting the bromide by an ether derivative of a second aromatic compound (Ar²-OH), thereby preparing the corresponding aryl ketone ether Ar1-CO-CHMe-OAr2; and
d) reducing the compound obtained from step c), thereby obtaining a polysphorin analog (i.e., Ar1-CHOH-CHMe-O-Ar2),
wherein
Ar1 is a phenyl, a diphenyl, a 2-benzimidazolyl, 2-quinolinyl, 1-isoquinolinyl, 2-indolyl or an indazolyl, the benzene ring thereof being optionally substituted by one to three substituents selected from the group consisting of a (C₁-C₃)alkyl, preferably a methyl or a fluorinated methyl (i.e., CH₂F, CHF₂ or CF₃), a (C₁-C₃)alkoxy, preferably OMe or a fluorinated methoxy (i.e., OCH₂F, OCHF₂ or OCF₃), a dimethylamino group, a fluoride, or by two adjacent substituents forming together -(CH₂)₃-, -O-CH₂-O-, -O-CF₂-O-, or -O-CHF-O-;
and Ar2 is a phenyl substituted by three substituents, two substituents in ortho of the main alkoxy group selected from the group consisting of OMe, F, CH₂F, CHF₂ or CF₃, OCH₂F, OCHF₂ and OCF₃, and one substituent in para of the ether bond selected from the group consisting of H, a C₁-C₆ alkyl, a C₂-C₆ alkenyl and a phenyl, optionally substituted by one to three substituents selected in the group consisting of a bromide, a C₁-C₃ alkyl or alkoxy, halogens, hydroxyl, cyano, amino, and nitro, preferably from the group consisting of H, a methyl, an allyl, a (E)-propenyl and a phenyl.
